Europäisches Patentamt

European Patent Office

Office européen des brevets

(19)

(11) Veröffentlichungsnummer: **0 317 509**
A2

(12) **EUROPÄISCHE PATENTANMELDUNG**

(21) Anmeldenummer: **88810762.0**

(22) Anmeldetag: **08.11.88**

(51) Int. Cl.⁴: **C 12 N 15/00**
**C 12 N 1/20, C 12 N 5/00,**
**A 01 H 1/00**

(30) Priorität: **16.11.87 CH 4453/87**

(43) Veröffentlichungstag der Anmeldung:
**24.05.89 Patentblatt 89/21**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Paszkowski, Jerzy, Dr.**
**Witikonerstrasse 231**
**CH-8053 Zürich (CH)**

**Baur, Markus**
**Vogesenstrasse 57**
**CH-4056 Basel (CH)**

**Potrykus, Ingo, Prof. Dr.**
**Im Stigler 54**
**CH-4312 Magden (CH)**

The applicant has filed a statement in accordance with Rule 28 (4) EPC (issue of a sample only to an expert). Accession number(s) of the deposit(s): DSM 4289.

(54) **Gezielter Einbau von Genen ins pflanzliche Genom.**

(57) Die vorliegende Erfindung betrifft ein neuartiges Verfahren, das basierend auf der homologen Rekombination von natürlichen oder artifiziell modifizierten Genen, Genfragmenten oder sonstigen nützlichen DNA-Sequenzen mit homologen DNA-Abschnitten innerhalb des pflanzlichen Genoms, den gezielten Einbau von genetischem Material an genau definierten Stellen in das Genom der Pflanze sowie eine zielgerichtete, vorhersagbare Modifikation von Genen innerhalb des pflanzlichen Genoms erlaubt. Weiterhin ermöglicht das erfindungsgemässe Verfahren eine gezielte Identifikation der Funktion von Einzelgenen innerhalb des Gesamtgenoms der Pflanzen. Ein weiterer Gegenstand dieser Erfindung betrifft nach diesem Verfahren erhältliche transgene Pflanzen und deren Nachkommen sowie die pflanzlichen Produkte davon.

Abbildung 1: Schema des 'Gene targeting' - Experiments

E.coli - Plasmid pUC19 DNA

Promotor- und Terminator-Region des 19S RNA Transkripts von Cauliflower Mosaik Virus (CaMV)
Promotor- und Terminator-Region des 35S RNA Transkripts von CaMV
Ende des Gen V von CaMV

APH(3') Protein kodierende Region von pABD1

APH(3')II Protein kodierende Region von pHP23Δ

EP 0 317 509 A2

**Beschreibung**

## Gezielter Einbau von Genen ins pflanzliche Genom

Die vorliegende Erfindung betrifft ein neuartiges Verfahren, das basierend auf der homologen Rekombination von natürlichen oder artifiziell modifizierten Genen, Genfragmenten oder sonstigen nützlichen DNA-Sequenzen mit homologen DNA-Abschnitten innerhalb des pflanzlichen Genoms, den gezielten Einbau von genetischem Material an genau definierten Stellen in das Genom der Pflanze und damit eine zielgerichtete, vorhersagbare Modifikation von Genen innerhalb des pflanzlichen Genoms erlaubt (in situ-Modifikation). Weiterhin ermöglicht das erfindungsgemässe Verfahren eine gezielte Identifikation der Funktion von Einzelgenen innerhalb des Gesamtgenoms der Pflanzen. Ein weiterer Gegenstand dieser Erfindung betrifft nach diesem Verfahren erhältliche transgene Pflanzen und deren Nachkommen sowie die pflanzlichen Produkte davon.

Voraussetzung für eine präzise genetische Manipulation von Pflanzen ist die genaue und vorhersagbare Veränderung des Pflanzengenoms durch den zielgerichteten Einbau von neuen Genen in das Genom von Pflanzenzellen, die dann zu Zell-, Gewebe- oder Kalluskulturen oder zu ganzen Pflanzen regeneriert werden und somit zu neuen Pflanzen führen, die nunmehr eine neue Eigenschaft aufweisen.

Die intensive Forschung auf dem Gebiet der rekombinanten DNA-Technologie bei Pflanzen hat in den letzten Jahren zu bedeutenden Fortschritten geführt. Dies gilt in erster Linie für die Herstellung transgener Pflanzen, wofür inzwischen eine Reihe von Gen-Transfer-Verfahren zur Verfügung stehen, die in vielen Laboratorien bereits routinemässig angewendet werden.

Zu den am besten etablierten und am häufigsten verwendeten Gen-Transfer-Verfahren gehört zweifellos das Agrobacterium-Transformationssystem.

Agrobacterium-Zellen besitzen auf ihrem Ti-Plasmid ein grosses DNA-Fragment, die sogenannte T-DNA-Region, das bei der Transformation pflanzlicher Zellen in das Pflanzengenom integriert wird.

Dieses natürliche Gen-Transfer-System kann nach Ausführung verschiedener Modifikationen als Gen-Vektor-System für die artifizielle Transformation von Pflanzen verwendet werden.

Darüberhinaus stehen noch andere effiziente Verfahren zur Einschleusung von genetischem Material in Pflanzen zur Verfügung, die zum Grossteil auf neueren Entwicklungen beruhen. Dazu gehören beispielsweise der direkte Gentransfer in Protoplasten, die Fusion DNA- haltiger, membranumhüllter Vesikel, sogenannter Liposomen, mit der Protoplastenmembran, die intranukleäre Mikroinjektion von Protoplasten sowie möglicherweise die Makroinjektion von genetischem Material in die meristematischen Bereiche des Blütenstandes.

Diese zuvor näher charakterisierten Gen-Transfer-Verfahren führen zwar zu einer Einschleusung von genetischem Material in die pflanzliche Zelle und zu einer Integration in das pflanzliche Genom; sie haben aber den entscheidenden Nachteil, dass der Einbau des eingeschleusten genetischen Materials in das nukleäre Genom der Pflanze ungerichtet und damit zufallsbedingt erfolgt, so dass die genomische Lokalisation der Fremd-DNA nicht vorherbestimmbar ist (Potrykus I et al., 1985; Wallroth M et al. 1986; Ambros PF et al., 1986).

Es ist aber andererseits bekannt, dass der Integrationsort innerhalb des pflanzlichen Genoms einen entscheidenden Einfluss auf die Effizienz und Stärke der Expression der integrierten Fremdgene hat. So können beispielsweise die Expressionsraten eines bestimmten integrierten Fremdgens in Abhängigkeit vom Integrationsort innerhalb des Genoms bei zwei verschiedenen unabhängig voneinander erzeugten transgenen Pflanzen beträchtlich differieren.

Die gleiche Abhängigkeit vom Integrationsort konnte auch im Zusammenhang mit der Gen-Regulation, insbesondere bei der Reaktion von integrierten Fremdgenen auf Induktions- und/oder Suppressor-Signale, beobachtet werden. Auch in diesem Fall findet man deutliche Unterschiede zwischen unabhängig voneinander erzeugten transgenen Pflanzen.

Diese Abhängigkeit vom Integrationsort kann beispielsweise dadurch überwunden werden, dass man eine in-situ Modifikation von Genen innerhalb des pflanzlichen Genoms durchführt.

Im Rahmen einer solchen in-situ Modifikation von bestimmten Zielgenen verbleibt das Gen an seiner natürlichen, angestammten Position innerhalb des Pflanzengenoms, sodass eine für das jeweilige Zielgen optimale Expression und Regulation gewährleistet ist.

Bis zum Zeitpunkt der vorliegenden Erfindung war aber kein Verfahren bekannt, das eine in-situ Modifikation von Genen innerhalb des pflanzlichen Genoms ermöglicht hätte.

Vor dem Hintergrund der allgemeinen Bemühungen auf dem Gebiet der pflanzlichen Gentechnologie um eine Steigerung der Effizienz der Regulation und Expression von eingeschleusten Fremdgenen in der pflanzlichen Zelle, muss es daher als eine vordringliche Aufgabe angesehen werden Verfahren zu entwickeln, die eine zielgerichtete Integration von Fremdgenen an definierten, vorherbestimmbaren Orten innerhalb des pflanzlichen Genoms erlauben und somit z.B. eine in-situ Modifikation von pflanzlichen Genen möglich machen.

Bei Hefen, einigen anderen Pilzen sowie bei dem Schleimpilz Dictyostelium discoideum (Hinnen H et al., 1978; Miller BL et al., 1987; De Lozanne A and Spudich SA., 1987) konnte die natürlicherweise sehr effiziente homologe Rekombination transformierender DNA für Untersuchungen über die natürliche genomische Umgebung der transformierten und modifizierten Gene eingesetzt werden sowie ausserdem für eine direkte Modifikation von Genen innerhalb des Genoms.

Dieses Verfahren lässt sich aufgrund der höheren Komplexizität auf die Verhältnisse bei taxonomisch höher stehenden Organismen dagegen nicht anwenden.

Bei Säugern beispielsweise findet man neben der erwähnten homologen Rekombination auch eine sehr effiziente, nicht homologe oder illegitime Rekombination von fremder DNA ins Genom, was einen gezielten Einbau von Fremd-DNA an einem bestimmten gewünschten Ort innerhalb des Genoms sehr schwierig macht.

Auch bei Pflanzen kann aufgrund der hohen Transformationsraten, die sich mit DNA Molekülen erzielen lassen, die keinerlei Homologien zu Abschnitten des pflanzlichen Genoms aufweisen (Shillito R et al., 1985; Negrutiu I et al., 1987), mit Sicherheit davon ausgegangen werden, dass der Anteil der illegitimen, nicht homologen Rekombination ebenfalls sehr hoch liegt.

Bis zum Zeitpunkt der vorliegenden Erfindung war es vollkommen unklar, ob in Pflanzen überhaupt eine homologe Rekombination existiert und falls ja, wie effizient diese arbeitet.

Folglich war es daher bisher auch nicht möglich, genetisches Material gezielt an genau definierten Stellen innerhalb des pflanzlichen Genoms zu integrieren.

Diese Aufgabe konnte jetzt im Rahmen der vorliegenden Erfindung überraschenderweise mit Hilfe einfacher Massnahmen gelöst werden.

Die vorliegende Erfindung betrifft ein Verfahren zur Herstellung von transgenen Pflanzen, das sich dadurch kennzeichnet, dass man ein Gen, ein Genfragment oder sonstige nützliche DNA-Sequenzen mit Hilfe der homologen Rekombination gezielt an einer gewünschten, vorherbestimmbaren Stelle in das pflanzliche Genom integriert.

Dabei soll der Begriff des pflanzlichen Genoms im Rahmen dieser Erfindung definitionsgemäss nicht auf das nukleäre Genom beschränkt bleiben, sondern auch die in den Mitochondrien und den Plastiden vorliegenden Genomabschnitte umfassen, die mit Hilfe des erfindungsgemässen Verfahrens ebenfalls einer gezielten Modifikation zugänglich sind.

Damit ist es im Rahmen dieser Erfindung jetzt erstmals möglich bestimmte Zielgene innerhalb des pflanzlichen Genoms gezielt und effizient in-situ zu modifizieren (gezielte Mutagenese).

Die in-situ Modifikation von Genen hat gegenüber den bisher praktizierten Verfahren zur Modifikation pflanzeneigener Gene, bei denen zunächst ein gewünschtes Gen isoliert, anschliessend in-vitro modifiziert und schliesslich wieder ins Genom reintegriert wird, entscheidende Vorteile.

Die Reintegration eines zuvor isolierten und in-vitro modifizierten Gens ins pflanzliche Genom erfolgt rein zufallsbedingt, sodass, in Abhängigkeit vom Integrationsort, Schwierigkeiten bei der Regulation und/oder Expression des integrierten Gens auftreten können. Bei der im Rahmen dieser Erfindung jetzt erstmals möglich gewordenen in-situ Modifikation verbleibt das Gen dagegen an seiner natürlichen, angestammten Position innerhalb des Pflanzengenoms, wodurch eine für das jeweilige Zielgen optimale Regulation und Expression gewährleistet wird.

Neben der zielgerichteten und damit sehr effizienten in-situ Modifikation von pflanzeneigenen Genen (gezielte Mutagenese), eröffnet das erfindungsgemässe Verfahren eine Reihe weiterer Anwendungsmöglichkeiten, wie z.B. den Einbau zusätzlicher Genkopien in Regionen mit bekannt hoher Expressionsrate sowie die Elimination und damit Ausschaltung von unerwünschten Genen u.a.m.

Im einzelnen ist es dabei mit Hilfe des erfindungsgemässen Verfahrens beispielsweise möglich, dominante oder semi-dominante Allele, die der Pflanze eine unter züchterischen und/oder kommerziellen Gesichtspunkten unerwünschte Eigenschaft verleihen, gegen rezessive Allele mit nützlichen und wünschenswerten Eigenschaften auszutauschen.

Eine weitere konkrete Einsatzmöglichkeit des erfindungsgemässen Verfahrens betrifft den gezielten Einbau eines gewünschten Gens ins pflanzliche Genom durch homologe Rekombination mit Genen oder Genfragmenten oder mit sonstigen nützlichen DNA-Sequenzen, die zuvor bereits mit Hilfe bekannter Verfahren artifiziell ins Pflanzengenom integriert wurden.

Das zuerst integrierte Gen oder Genfragment bzw. die zuerst integrierte DNA-Sequenz kann dabei z.B. als Sondenmolekül eingesetzt werden, mit dessen Hilfe es möglich ist, Regionen innerhalb des pflanzlichen Genoms aufzuspüren, die vorteilhafte Eigenschaften aufweisen, wie z.B. eine gute Expression oder eine gute Regulierbarkeit des eingebauten Gens.

Mit Hilfe des erfindungsgemässen Verfahrens kann dann ein gewünschtes Gen oder Genfragment oder eine sonstige nützliche DNA-Sequenz sehr einfach und gezielt in diesen, mit Hinblick auf die Genexpression oder die Regulierbarkeit bevorzugten Bereich innerhalb des Pflanzengenoms via homologer Rekombination dirigiert werden.

Besonders bevorzugt für eine Verwendung als Sondenmoleküle sind Gene oder Genfragmente bzw. sonstige nützliche DNA-Sequenzen, die sich durch eine leichte Selektionierbarkeit auszeichnen, wie z.B. Gene, die zu phenotypischen Selektionsmerkmalen in der transformierten Zelle oder Pflanze führen, insbesondere zu Resistenzen gegenüber Antibiotika oder gegenüber bestimmten Herbiziden.

In gleicher Weise ist es möglich Gene oder Genfragmente oder sonstige nützliche DNA-Sequenzen mit Genen oder Genfragmenten zu koppeln, die zuvor bereits mit Hilfe bekannter Verfahren artifiziell ins pflanzliche Genom integriert wurden.

So kann beispielsweise ein Gen, das eine wünschenswerte Eigenschaft kodiert, wie z.B. eine Insektenresistenz, mit einem zweiten, zuvor bereits artifiziell ins Genom einer Zielpflanze integrierten Gen, das beispielsweise eine Herbizidresistenz kodiert, gekoppelt werden.

Das erfindungsgemässe Verfahren ist aber nicht auf den Austausch bzw. die Kopplung von Genen oder sonstigen DNA-Sequenzen mit zuvor artifiziell ins pflanzliche Genom integrierten Genen oder DNA-Sequenzen beschränkt.

Es ist selbstverständlich mit Hilfe des erfindungsgemässen Verfahrens in gleicher Weise möglich, gewünschte Gene oder Genfragmente sowie andere nützliche DNA-Sequenzen mit natürlicherweise im Pflanzengenom bereits vorliegenden Genen auszutauschen oder an diese zu koppeln.

Dabei kann man beispielsweise ein unter züchterischen Gesichtspunkten gewünschtes und nützliches Gen mit einem Markergen koppeln, das sich in den Folgegenerationen aufgrund seiner phänotypischen Merkmalsausprägung leicht verfolgen lässt.

Auf diese Weise ist es für den Züchter sehr einfach möglich, ein gewünschtes Gen durch die Folgegenerationen hindurch zu verfolgen, indem er sich an dem phänotypischen Markergen orientiert.

Ein weiterer Gegenstand der vorliegenden Erfindung betrifft daher rekombinante DNA Moleküle, die aufgrund ihrer spezifischen Konstruktion in der Lage sind Gene oder DNA-Sequenzen, die gegebenenfalls für neue und wünschenswerte Eigenschaften kodieren, mit Hilfe der homologen Rekombination gezielt an definierten Stellen innerhalb des pflanzlichen Genoms zu integrieren.

Weiterhin umfasst von der vorliegenden Erfindung sind ferner transgene Pflanzen mit neuen und/oder verbesserten Eigenschaften, die mit besagtem rekombinanten DNA-Molekül transformiert worden sind.

Die vorliegende Erfindung betrifft ausserdem transgene Pflanzen, welche aus transformierten Pflanzenzellen, die besagtes rekombinantes DNA Molekül enthalten, regeneriert werden sowie deren Samen, darüberhinaus die Nachkommen von transgenen Pflanzen sowie Mutanten und Varianten davon.

Die vorliegende Erfindung umfasst ebenso chimäre genetische Konstruktionen, weiterhin Klonierungsvehikel und Wirtsorganismen sowie Methoden zur gezielten Uebertragung wünschenswerter Eigenschaften auf Pflanzen.

In der folgenden Beschreibung werden eine Reihe von Ausdrücken verwendet, die in der rekombinanten DNA Technologie, sowie in der Pflanzengenetik gebräuchlich sind.

Um ein klares und einheitliches Verständnis der Beschreibung und der Ansprüche sowie des Umfangs, der den besagten Ausdrücken zukommen soll, zu gewährleisten, werden die folgenden Definitionen aufgestellt:

Heterologe(s) Gen(e) oder DNA:
Eine DNA Sequenz, die für ein spezifisches Produkt oder Produkte kodiert oder eine biologische Funktion erfüllt und die von einer anderen Spezies stammt als derjenigen, in welche das besagte Gen eingeschleust wird; besagte DNA Sequenz wird auch als Fremdgen oder Fremd-DNA bezeichnet.

Homologe(s) Gen(e) oder DNA:
Eine DNA Sequenz, die für ein spezifisches Produkt oder Produkte kodiert oder eine biologische Funktion erfüllt und die aus der gleichen Spezies stammt, in welche das besagte Gen eingeschleust wird.

Synthetische(s) Gen(e) oder DNA:
Eine DNA-Sequenz, die für ein spezifisches Produkt oder Produkte kodiert oder eine biologische Funktion erfüllt und die auf synthetischem Wege hergestellt sind.

Pflanzen-Promotor:
Eine Kontrollsequenz der DNA Expression, die die Transkription jeder beliebigen homologen oder heterologen DNA Gensequenz in einer Pflanze gewährleistet, sofern besagte Gensequenz in operabler Weise mit einem solchen Promotor verknüpft ist.

Terminations-Sequenz:
DNA-Sequenz am Ende einer Transkriptions-Einheit, die das Ende des Transkriptionsvorganges signalisiert.

Ueberproduzierender Pflanzen-Promotor (OPP):
Pflanzen-Promotor, der in der Lage ist, in einer transgenen Pflanzenzelle die Expression einer jeden in operabler Weise verknüpften funktionalen Gensequenz(en) in einem Ausmass (gemessen in Form der RNA oder der Polypeptidmenge) zu bewirken, das deutlich höher liegt, als dies natürlicherweise in Wirtszellen, die nicht mit besagtem OPP transformiert sind, beobachtet wird.

3'/5' nicht-translatierte Region:
Stromabwärts/stromaufwärts der kodierenden Region gelegene DNA-Abschnitte, die zwar in mRNA transkribiert, nicht aber in ein Polypeptid übersetzt werden. Diese Region enthält regulatorische Sequenzen, wie z.B die Ribosomenbindungsstelle (5') oder das Polyadenylierungssignal (3').

Pflanzliches Material:
in Kultur oder als solches lebensfähige pflanzliche Teile wie Protoplasten, Zellen, Kallus, Gewebe, Embryonen, Pflanzenorgane, Knospen, Samen, u.a. sowie ganze Pflanzen.

Pflanzen-Zelle:
Strukturelle und physiologische Einheit der Pflanze, bestehend aus einem Protoplasten und einer Zellwand.

Protoplast:
aus Pflanzenzellen oder -geweben isolierte zellwandlose "nackte" Pflanzenzelle mit der Potenz zu einem Zellklon oder einer ganzen Pflanze zu regenerieren.

Zellklon:
aus einer Zelle durch fortlaufende Mitosen entstandene Population von genetisch gleichen Zellen.

Pflanzengewebe:
Eine Gruppe von Pflanzenzellen, die in Form einer strukturellen und funktionellen Einheit organisiert sind.

Pflanzenorgan:
Eine strukturelle und funktionelle Einheit zusammengesetzt aus mehreren Geweben, wie beispielsweise Wurzel, Stamm, Blatt oder Embryo.

DNA-Expressions-Vektor:
Klonierungs-Vehikel, wie z.B. ein Plasmid oder ein Bakteriophage, das alle Signal-Sequenzen enthält, die für die Expression einer inserierten DNA in einer geeigneten Wirtszelle notwendig sind.

DNA-Transfer-Vektor:
Transfer-Vehikel, wie z.B. ein Ti-Plasmid oder ein Virus, das die Einschleusung von genetischem Material in eine geeignete Wirtszelle ermöglicht.

homologe Rekombination:
reziproker Austausch von Stücken zwischen homologen, doppelsträngigen DNA-Molekülen.

Mutanten, Varianten transgener Pflanzen:
Spontan oder aber artifiziell, unter Anwendung bekannter Verfahrensmassnahmen, wie z.B. UV-Behandlung, Behandlung mit mutagenen Agentien etc. entstandener Abkömmling einer transgenen Pflanze, der noch die erfindungswesentlichen Merkmale und Eigenschaften der transgenen Ausgangspflanze aufweist, welche diese aufgrund der Transformation mit exogener DNA erhalten hat.

Im Rahmen der vorliegenden Erfindung ist es jetzt erstmals gelungen, ein Verfahren zu entwickeln, das es ermöglicht bei Pflanzen genetisches Material gezielt an definierten vorherbestimmten Positionen innerhalb des pflanzlichen Genoms zu integrieren und dort zu exprimieren.

Das erfindungsgemässe Verfahren beruht auf einer gezielten Integration von genetischem Material in das Genom der Pflanze mit Hilfe der homologen Rekombination von natürlichen oder artifiziell modifizierten und/oder synthetischen Genen oder Genfragmenten oder sonstigen nützlichen DNA-Sequenzen mit homologen DNA-Abschnitten innerhalb des pflanzlichen Genoms, wobei besagte homologe DNA-Abschnitte ein natürlicher Bestandteil des Pflanzengenoms sind oder aber gegebenenfalls zuvor mit Hilfe bekannter Verfahrensmassnahmen artifiziell in das Pflanzengenom eingebaut werden.

Einen wesentlichen Bestandteil der vorliegenden Erfindungen bilden daher rekombinante DNA-Moleküle, die aufgrund ihrer spezifischen Konstruktion den gezielten Einbau von Genen in das Genom einer Zielpflanze mit Hilfe der homologen Rekombination ermöglichen.

Insbesondere betrifft die vorliegende Erfindung rekombinante DNA Moleküle, die eine zielgerichtete Integration von Genen, Genfragmenten oder sonstigen nützlichen DNA-Sequenzen an genau definierten Stellen innerhalb des pflanzlichen Genoms ermöglichen und die sich dadurch kennzeichnen, dass sie die zu integrierende DNA enthalten, und dass besagte DNA in einem Masse Homologien zu entsprechenden DNA Regionen innerhalb des pflanzlichen Genoms aufweist oder aber von solchen homologen DNA-Sequenzen flankiert wird, dass bei der Transformation der die homologen DNA Regionen enthaltenden pflanzlichen Zelle eine zielgerichtete Integration besagter zu integrierender DNA an genau definierten Stellen innerhalb des pflanzlichen Genoms durch homologe Rekombination stattfindet.

Es handelt sich dabei bevorzugterweise um rekombinante DNA Moleküle, die aus einer zu integrierenden DNA-Sequenz bestehen, welche gegebenenfalls in operabler Weise mit in der pflanzlichen Zelle funktionsfähigen Expressionssignalen sowie mit flankierenden DNA-Abschnitten, die Homologien zu einer bestimmten Region innerhalb des pflanzlichen Genoms aufweisen, verknüpft ist. In erster Linie betrifft die vorliegende Erfindung rekombinante DNA Moleküle, die sich dadurch kennzeichnen, dass

a) besagte rekombinanate DNA Moleküle ein Strukturgen besitzen, das für eine nützliche und wünschenswerte Eigenschaft kodiert

b) besagtes Strukturgen gegebenenfalls in operabler Weise mit in pflanzlichen Zellen funktionsfähigen Expressionssignalen verknüpft ist

c) besagte funktionelle Einheit, bestehend aus Strukturgen und gegebenenfalls aus Expressionssignalen, in einer Weise von DNA-Sequenzen flankiert wird, die in einem Masse Homologien zu entsprechenden DNA-Regionen innerhalb des pflanzlichen Genoms aufweisen, dass bei der Transformation der die besagte homologe Region enthaltenden pflanzlichen Zelle eine gezielte Integration der von homologen DNA-Sequenzen flankierten Gensequenz an definierten Stellen innerhalb des pflanzlichen Genoms durch homologe Rekombination stattfindet.

Neben Strukturgenen können auch beliebige andere Gene oder Genfragmente sowie DNA-Sequenzen verwendet werden.

Ebenso umfasst vom breiten Konzept dieser Erfindung sind genetische Konstruktionen, bestehend aus Genen oder Genfragmenten oder sonstigen nützlichen DNA-Sequenzen, die gegebenenfalls unter der Kontrolle von in pflanzlichen Zellen funktionsfähigen Expressionssignalen stehen und die eine genügend grosse Homologie zu entsprechenden DNA Regionen innerhalb des pflanzlichen Genoms aufweisen, sodass ein direkter Austausch dieser Gene oder Genfragmente oder sonstiger nützlicher DNA-Sequenzen gegen besagte homologe genomische DNA vermittels der homologen Rekombination erfolgen kann.

Neben doppelsträngiger DNA kann in dem erfindungsgemässen Verfahren auch einzelsträngige DNA sowie teilweise einzelsträngige DNA eingesetzt werden.

Darüberhinaus besteht die Möglichkeit DNA/Protein-Komplexe zu verwenden, in denen die zu integrierende Ziel-DNA mit einem bestimmten funktionalen Protein assoziiert ist.

An dieser Stelle seien beispielhaft einige DNA/Protein-Komplexe genannt, wie z.B. ein DNA/Chromatinbindungsprotein-Komplex, ein DNA/Pflanzenvirus-Protein-Komplex oder ein DNA/Protein-Komplex unter Einbeziehung heterologer Proteine wie z.B. von Rekombinationsproteinen aus Bakterien oder Hefe (z.B. RecA, B, C, D).

Für die Verwendung in dem erfindungsgemässen Verfahren sind in erster Linie alle die Gene geeignet, die in der pflanzlichen Zelle exprimiert werden und die der Pflanze eine nützliche und/oder gewünschte Eigenschaft verleihen, wie z.B. eine erhöhte Resistenz oder Toleranz gegenüber Pathogenen (z.B. phytophatogene Insekten, Pilze, Bakterien, Viren usw.) gegenüber Herbiziden, Insektiziden oder anderen Bioziden, gegenüber klimatischen Einflüssen und lokalen Besonderheiten (z.B. Hitze, Kälte, Wind, Trockenheit, Feuchtigkeit, besondere extreme Bodenverhältnisse, osmotischer Stress usw.) oder aber eine erhöhte Bildung von Reserve- und Lagerstoffen in Blättern, Samen, Knollen, Wurzeln, Stengeln usw.

Ebenso werden von der vorliegenden Erfindung Gene umfasst, die für pharmazeutisch akzeptable Wirksubstanzen, wie z.B. Alkaloide, Steroide, Hormone, Immunmodulatoren, und andere physiologisch aktive Substanzen kodieren.

Neben natürlicherweise vorkommenden Genen, die für eine nützliche und wünschenswerte Eigenschaft kodieren, können im Rahmen dieser Erfindung auch Gene verwendet werden, die zuvor mit Hilfe chemischer oder gentechnologischer Methoden gezielt modifiziert wurden.

Weiterhin sind von dem breiten Konzept der vorliegenden Erfindung auch solche Gene umfasst, die vollständig durch chemische Synthese hergestellt werden.

Beispielhaft seien hier die pflanzlichen Speicherprotein-Gene erwähnt. Mögliche Modifikationen der Proteingene beziehen sich in erster Linie auf deren Aminosäurezusammensetzung, die bekanntermassen in den meisten Fällen nur von geringem ernährungsphysiologischem Wert ist. So weist beispielsweise das Zein-Speicherprotein von Mais, dessen Protein-kodierende DNA-Sequenz bekannt ist (Wienand K et al, Mol. Gen-Genet., 182: 440 - 444, 1981) einen hohen Prolin-Gehalt auf, während der Lysin- und Tryptophan-Anteil sehr gering ist.

Durch Austausch von pflanzeneigenen Zein-Genen mit einem zuvor modifizierten, jetzt für ein Lysin und/oder Tryptophan-reicheres Protein oder ein in anderer Weise verbessertes Zein-Protein kodierenden Gen unter Verwendung des erfindungsgemässen Verfahrens, ist es nunmehr möglich den ernährungsphysiologischen Wert bestimmter Zielpflanzen gezielt zu verbessern.

Darüberhinaus kann zusätzlich der hohe Background an pflanzeneigenen Speicherproteinen mit geringem ernährungsphysiologischem Wert durch die gezielte Integration von Störgenen, welche besagte pflanzeneigenen Gene inaktivieren, verringert werden.

Ein Beispiel für ein solches Störgen ist das opaque-2 Gen von Mais, welches die Expression der Zeinproteine unterbindet.

Weiterhin geeignet für die Verwendung in dem erfindungsgemässen Verfahren sind sonstige nützliche DNA-Sequenzen, insbesondere nicht-kodierende DNA-Sequenzen mit regulatorischen Funktionen.

Für die Verwendung in dem erfindungsgemässen Verfahren kommen somit sowohl homologe als auch heterologe Gen(e) oder DNA sowie synthetische Gen(e) oder DNA gemäss der im Rahmen der vorliegenden Erfindung gemachten Definition in Betracht.

Die zu integrierenden DNA-Sequenzen können dabei ausschliesslich aus genomischer, aus cDNA bzw. synthetischer DNA konstruiert sein. Eine andere Möglichkeit besteht in der Konstruktion von hybriden DNA-Sequenzen, bestehend sowohl aus cDNA als auch aus genomischer DNA und/oder synthetischer DNA.

In diesem Fall kann die cDNA aus demselben Gen oder DNA-Abschnitt stammen wie die genomische DNA oder aber, sowohl die cDNA, wie auch die genomische DNA können aus verschiedenen Genen oder DNA-Abschnitten stammen. In jedem Fall können aber sowohl die genomischen DNA und/ oder die cDNA, jede für sich, aus dem gleichen oder aus verschiedenen Genen oder DNA-Abschnitten hergestellt sein.

Wenn die DNA-Sequenz Anteile von mehr als einem Gen oder DNA-Abschnitt beinhaltet, können diese entweder von ein und demselben Organismus, von mehreren Organismen, die verschiedenen Stämmen, oder Varietäten derselben Art oder verschiedenen Spezies derselben Gattung angehören, oder aber von Organismen, die mehr als einer Gattung derselben oder einer anderen taxonomischen Einheit angehören, abstammen.

Um die Expression eines Strukturgens in der pflanzlichen Zelle zu gewährleisten, können die kodierenden Gensequenzen gegebenenfalls zunächst in operabler Weise mit in pflanzlichen Zellen funktionsfähigen Expressions-Sequenzen verknüpft werden.

Die hybriden Genkonstruktionen der vorliegenden Erfindung enthalten somit in der Regel neben dem (den) Strukturgen(en) Expressions-Signale, die sowohl Promoter- und Terminator-Sequenzen als auch weitere regulatorische Sequenzen der 3′ und 5′ nicht translatierten Regionen miteinschliessen.

Jeder Promotor und jeder Terminator, der in der Lage ist, eine Induktion der Expression einer kodierenden DNA-Sequenz (Strukturgen) zu bewirken, kann als Bestandteil der chimären Gensequenz verwendet werden. Besonders geeignet sind Expressionssignale, die aus Genen von Pflanzen oder Pflanzenviren stammen. Beispiele geeigneter Promotoren und Terminatoren sind z.B. solche der Nopalin-Synthase-Gene (nos), der Octopin-Synthase-Gene (ocs) sowie der Cauliflower Mosaik Virus Gene (CaMV).

Bevorzugt im Rahmen dieser Erfindung sind die 35S und 19S Expressions-Signale des CaMV Genoms, die mit Hilfe molekularbiologischer Methoden, wie sie z.B. bei Maniatis et al., 1982 beschrieben sind, aus besagtem Genom isoliert und mit der kodierenden DNA-Sequenz verknüpft werden können.

Als Ausgangsmaterial für die 35S-Transkriptionskontroll-Sequenzen kann erfindungsgemäss z.B. das ScaI-Fragment des CaMV Stammes "S", das die Nukleotide 6808-7632 der Genkarte umfasst (Frank G et al., 1980), verwendet werden.

Die 19S Promoter- und 5′ nicht-translatierte Region befindet sich auf einem Genomfragment zwischen der PstI Stelle (Position 5386) und der HindIII-Stelle (Position 5850) der CaMV Genkarte (Hohn et al., 1982). Die entsprechende Terminator- und 3' nichttranslatierte Region liegt auf einem EcoRV/BglII-Fragment zwischen Position 7342 und 7643 des CaMV Genoms.

Weiterhin bevorzugt im Rahmen dieser Erfindung sind die Expressionssignale des CaMV Stammes CM 1841, dessen komplette Nukleotidsequenz bei Gardner RC et al, 1981 beschrieben ist.

Ein weiterer wirksamer Vertreter eines Pflanzenpromotors, der Verwendung finden kann, ist ein überproduzierender Pflanzenpromotor. Diese Art von Promotoren sollte, sofern sie in operabler Weise mit der Gensequenz, welche für ein gewünschtes Genprodukt kodiert verknüpft ist, in der Lage sein, die Expression besagter Gensequenz zu vermitteln.

Ueberproduzierende Pflanzenpromotoren, die im Rahmen der vorliegenden Erfindung zur Anwendung kommen können, schliessen den Promotor der kleinen Untereinheit (small subunit; ss) der Ribulose-1,5-bisphosphat-Carboxylase aus Sojabohnen [Berry-Lowe et al., J. Molecular and App. Gen., 1: 483-498 (1982)] sowie den Promotor des Chlorophyll-a/b-Bindungsproteins ein. Diese beiden Promotoren sind dafür bekannt, dass sie in eukaryontischen Pflanzenzellen durch Licht induziert werden [siehe z.B. Genetic Engineering of Plants, an Agricultural Perspective, A. Cashmore, Plenum, New York 1983, Seite 29-38; Coruzzi G. et al., The Journal of Biological Chemistry, 258: 1399 (1983) und Dunsmuir, P. et al., Journal of Molecular and Applied Genetics, 2: 285 (1983)].

Die somit gebildete funktionelle Einheit bestehend aus einem Gen, einer hybriden Genkonstruktion oder sonstigen nützlichen DNA-Sequenzen, die gegebenenfalls unter der regulatorischen Kontrolle von in pflanzlichen Zellen aktiven Expressionssignalen stehen, wird anschliessend mit DNA-Abschnitten flankiert, die in einem Masse Homologien zu entsprechenden DNA-Regionen innerhalb des pflanzlichen Genoms aufweisen, dass bei der Transformation der die besagte homologen Region enthaltenden pflanzlichen Zelle eine gezielte Integration der von homologen DNA-Sequenzen flankierten Gensequenz an definierten Stellen innerhalb des pflanzlichen Genoms durch homologe Rekombination stattfindet.

Durch die Wahl geeigneter flankierender DNA-Sequenzen, die hinreichend grosse Homologien zu entsprechenden Abschnitten innerhalb des pflanzlichen Genoms aufweisen und damit einen Austausch von genetischem Material via homologer Rekombination ermöglichen, ist es nunmehr erstmals möglich, Gene gezielt an vorherbestimmten Stellen des Pflanzengenoms zu integrieren und gegebenenfalls zu exprimieren.

Das Ausmass der für einen Austausch via homologer Rekombination benötigten Homologie zwischen flankierender DNA und der entsprechenden genomischen DNA-Region ist von verschiedenen Parametern abhängig, wie z.B. von der spezifischen Chromatinstruktur u.a., und muss daher in Abhängigkeit von den verwendeten DNA-Sequenzen jeweils den entsprechenden Bedürfnissen angepasst werden.

Für die Verwendung als flankierende DNA sind im Rahmen dieser Erfindung in erster Linie DNA-Sequenzen geeignet, die Homologien zu solchen pflanzeneigenen Genen und/oder Genomabschnitten aufweisen, deren Nukleotidsequenzen ganz oder teilweise bekannt sind, wobei solche Gene oder Genomabschnitte bevorzugt sind, die in Bereichen lokalisiert sind, die natürlicherweise bereits eine hohe Expressionseffizienz aufweisen.

Beispielhaft seien hier die pflanzlichen Speicherproteingene erwähnt, - ohne dadurch den Erfindungsgegenstand zu limitieren - von denen bekannt ist, dass sie, abhängig vom Entwicklungsstadium der Pflanze, sehr hohe Expressionsraten erreichen.

Weitere Gene, deren Nukleotidsequenz zumindest bereits zum Teil bekannt ist und die daher in dem erfindungsgemässen Verfahren einsetzbar sind, umfassen z.B. das Nitrat-Reduktase Gen, das psbA Gen (Gressel J., Oxford Surv. Plant Mol. Cell Biol., 2: 321 - 328, 1985; Zurawski G. et al., Proc. Natl. Acad. Sci, USA, 79: 7699 - 7703, 1982), das Gen der kleinen (ss) Untereinheit der Ribulose-1,5-bisphosphat-Carboxylase, sowie das Alpha-Amylase und das Sucrose-Synthetase Gen. Neben pflanzenspezifischen Genen können im Rahmen dieser Erfindung auch alle diejenigen Gene verwendet werden, die aufgrund eines stark ausgeprägten Konservatismus im Verlaufe der Evolution grössere Bereiche besitzen, die Homologien zu den entsprechenden pflanzenspezifischen Genen aufweisen.

Dazu gehören beispielsweise die Histon-Gene, die Actin-Gene sowie die Glutathion-S-transferase-Gene der Mammalier, desweiteren verschiedene mikrobielle Gene, wie z.B. die Tryphophan- und Glutamin-Synthase-Gene.

Diese beispielhafte Aufzählung möglicher und für die Anwendung in dem erfindungsgemässen Verfahren geeigneter Gene dient lediglich der besseren Illustrierung der vorliegenden Erfindung und soll den Erfindungsgegenstand in keiner Weise einschränken.

Weiterhin sind für die Verwendung in dem erfindungsgemässen Verfahren solche DNA-Sequenzen geeignet, die Homologien zu Genen oder Genfragmenten oder zu sonstigen nützlichen DNA-Sequenzen aufweisen, welche zuvor mit Hilfe bekannter Verfahrensmassnahmen artifiziell ins Genom einer Zielpflanze integriert wurden und deren Nukleotidsequenzen ganz oder teilweise bekannt sind. Besonders bevorzugt sind solche DNA-Sequenzen, die in Bereichen lokalisiert sind, die natürlicherweise eine hohe Expressionseffizienz gewährleisten.

In einer spezifischen Ausführungsform der vorliegenden Erfindung handelt es sich bei der zuintegrierenden DNA und der bereits im pflanzlichen Genom vorliegenden artifiziell integrierten DNA um sich gegenseitig komplementierende DNA-Sequenzen, die nach erfolgter homologer Rekombination eine funktionale Einheit innerhalb des Pflanzengenoms bilden.

Bei besagter funktioneller Einheit kann es sich um ein Strukturgen handeln, das ein wünschenswertes und nützliches Proteinprodukt kodiert oder aber um eine Kontrollsequenz, die eine spezifische regulatorische Funktion innerhalb des pflanzlichen Genoms erfüllt.

Besonders bevorzugt im Rahmen dieser Erfindung sind sich gegenseitig komplementierende DNA-Sequenzen, die nach erfolgter Rekombination ein funktionsfähiges Strukturgen bilden, das einen spezifischen phänotypischen Marker oder ein sonstiges leicht detektierbares Proteinprodukt kodiert.

Somit ist es im Rahmen dieser Erfindung nunmehr möglich, durch die Herstellung entsprechender hybrider Genkonstruktionen in der zuvor beschriebenen Weise gezielte vorhersagbare Modifikationen von pflanzeneigenen Genen innerhalb des pflanzlichen Genoms vorzunehmen.

Einen wesentlichen Bestandteil der vorliegenden Erfindung bildet somit ein Verfahren zur Herstellung transgener Pflanzen, das sich dadurch kennzeichnet, dass man pflanzliches Material mit einem der zuvor beschriebenen rekombinanten DNA Moleküle transformiert, wobei die in besagtem rekombinanten DNA Molekül enthaltenen Gene, hybriden Genkonstruktionen oder sonstigen nützlichen DNA Sequenzen durch homologe Rekombination gezielt an einer gewünschten vorherbestimmbaren Stelle in das pflanzliche Genom integriert werden.

In einem ersten Verfahrensschritt werden zunächst die zuvor beschriebenen rekombinanten DNA Moleküle, die beispielsweise aus einem oder mehreren Strukturgenen, die für ein nützliches und wünschenswertes Genprodukt kodieren und die in operabler Weise mit in der pflanzlichen Zelle funktionsfähigen Expressionssignalen verknüpft sind, bestehen sowie aus flankierenden Sequenzabschnitten, die Homologien zu pflanzeneigenen Genomabschnitten aufweisen, konstruiert und in geeignete Klonierungsvektoren eingespleisst, die in der Lage sind mit Hilfe der homologen Rekombination wieder ein funktionsfähiges Gen zu bilden.

Neben Strukturgenen können auch beliebige andere wünschenswerte Gene oder Genfragmente oder sonstige nützliche DNA-Sequenzen verwendet werden.

Als Klonierungsvektoren verwendet man im allgemeinen Plasmid- oder Virus-(Bakteriophage)-Vektoren mit Replikations- und Kontrollsequenzen, die von Spezies stammen, die mit der Wirtszelle kompatibel sind.

Der Klonierungsvektor trägt in der Regel einen Replikationsursprung, ausserdem spezifische Gene, die zu phaenotypischen Selektionsmerkmalen in der transformierten Wirtszelle führen, insbesondere zu Resistenzen gegenüber Antibiotika oder gegenüber bestimmten Herbiziden. Die transformierten Vektoren können anhand dieser phenotypischen Marker nach einer Transformation in einer Wirtszelle selektiert werden.

Selektierbare phaenotypische Marker, die im Rahmen dieser Erfindung Anwendung finden können, umfassen beispielsweise, ohne das dies eine Limitierung des Erfindungsgegenstandes darstellt, Resistenzen gegen Ampicillin, Tetracyklin, Hygromycin, G418, Kanamycin und Neomycin.

Als Wirtszellen kommen im Rahmen dieser Erfindung Prokaryonten in Frage, einschliesslich bakterieller Wirte, wie z.B. A.tumefaciens, E.coli, S.typhimurium und Serratia marcescens, sowie Cyanobakterien. Ferner können auch eukaryontische Wirte wie Hefen, mycelbildende Pilze und Pflanzenzellen im Rahmen dieser Erfindung verwendet werden.

Das Einspleissen der erfindungsgemässen chimären Genkonstruktion in einen geeigneten Klonierungsvektor erfolgt mit Hilfe von Standardmethoden wie sie z.B. bei Maniatis et al., 1982 beschrieben sind.

Dabei wird in der Regel der Vektor und die einzuspleissende DNA-Sequenz zunächst mit geeigneten Restriktions-Enzymen geschnitten. Geeignete Restriktionsenzyme sind z.B. solche, die Fragmente mit glatten Enden liefern, wie z.B. SmaI, HpaI und EcoRV, oder aber Enzyme, die kohesive Enden bilden, wie z.B. EcoRI, SacI und BamHI.

Sowohl Fragmente mit glatten Enden wie auch solche mit kohesiven Enden, die einander komplementär sind, können mit Hilfe geeigneter DNA-Ligasen wieder zu einem einheitlichen durchgehenden DNA-Molekül verknüpft werden.

Glatte Enden können auch durch Behandlung von DNA-Fragmenten, die überhängende kohäsive Enden aufweisen, mit dem Klenow-Fragment der E.coli DNA-Polymerase durch Auffüllen der Lücken mit den entsprechenden komplementären Nucleotiden hergestellt werden.

Anderseits lassen sich auch kohäsive Enden künstlich herstellen, z.B. durch Anfügen komplementärer homopolymerer Schwänze an die Enden einer gewünschten DNA-Sequenz sowie des geschnittenen Vektormoleküls unter Verwendung einer terminalen Deoxynukleotidyl-Transferase oder aber durch Anfügen synthetischer Oligonukleotid-Sequenzen (Linker), die eine Restriktionsschnittstelle tragen und anschliessendes Schneiden mit dem entsprechenden Enzym.

Der Klonierungsvektor und die mit diesem Vektor transformierte Wirtszelle werden erfindungsgemäss dazu verwendet, die Kopienzahl des Vektors zu erhöhen. Mit einer erhöhten Kopienzahl ist es möglich, den Vektor, der die erfindungsgemässe hybride Genkonstruktion trägt, zu isolieren und z.B. für die Einschleusung der chimären Gensequenz in die pflanzliche Zelle zu verwenden.

Ein wesentlicher Bestandteil der vorliegenden Erfindung betrifft daher die Konstruktion von Plasmiden, die besagte chimäre Genkonstruktionen, bestehend aus einem oder mehreren Strukturgenen oder sonstigen wünschenswerten Genen bzw. Genfragmenten oder sonstigen nützlichen DNA-Sequenzen, die gegebenenfalls in operabler Weise mit in pflanzlichen Zellen funktionsfähigen Expressionssignalen verknüpft sind, sowie aus flankierenden DNA-Sequenzen, die Homologien zu pflanzeneigenen Genomabschnitten besitzen, aufweisen.

In einem weiteren Verfahrensschritt können diese Plasmide dazu verwendet werden, die zuvor beschriebene chimäre genetische Konstruktion, die gegebenenfalls ein Strukturgen enthält, welches für ein gewünschtes Genprodukt kodiert, in die pflanzliche Zelle einzuschleusen und in das Pflanzengenom zu integrieren.

Ein weiterer Gegenstand der vorliegenden Erfindung betrifft daher die Herstellung von pflanzlichen Rezipienten-Zellen, welche besagtes Strukturgen oder sonstige wünschenswerte Gene bzw. Genfragmente oder sonstige nützliche DNA-Sequenzen in ihr Genom eingebaut enthalten.

Die Einschleusung der chimären genetischen Konstruktion erfolgt vorzugsweise in pflanzliche Protoplasten mit Hilfe bekannter Gen-Transfer-Verfahren.

Es existieren mittlerweile eine Reihe von sehr effizienten Verfahren für die Einführung von DNA in Pflanzenzellen, basierend auf der Verwendung von Gentransfer-Vektoren oder auf direkten Gentransfer-Verfahren.

Eine Möglichkeit besteht beispielsweise darin, dass man Pflanzenzellen mit Viren oder mit Agrobacterium in Kontakt bringt. Dies kann durch Infektion sensitiver Pflanzenzellen oder durch Co-Cultivierung von Protoplasten, die sich aus Pflanzenzellen ableiten, bewerkstelligt werden. Das Cauliflower Mosaik Virus (CaMV) kann im Rahmen dieser Erfindung ebenso als Vektor für die Einschleusung der erfindungsgemässen chimären genetischen Konstruktion in die Pflanze verwendet werden (Hohn et al., in "Molecular Biology of Plant Tumors", Academic Press, New York, 1982, Seite 549-560; Howell, US-Patent Patent No. 4,407,956). Das gesamte virale DNA-Genom von CaMV wird dabei in ein bakterielles Elternplasmid integriert unter Ausbildung eines rekombinanten DNA Moleküls, das in Bakterien vermehrt werden kann. Nach erfolgter Klonierung wird das rekombinante Plasmid mit Hilfe von Restriktionsenzymen entweder zufällig oder an ganz spezifischen nicht essentiellen Stellen innerhalb des viralen Teils des rekombinanten Plasmids gespalten, z.B. innerhalb des Gens, das für die Uebertragbarkeit des Virus durch Blattläuse kodiert, um die hybride Genkonstruktion einbauen zu können.

Ein kleines Oligonukleotid, ein sog. Linker, der eine einzige, spezifische Restriktionserkennungsstelle besitzt, kann ebenfalls integriert werden. Das so modifizierte rekombinante Plasmid wird erneut kloniert und durch Einspleissen der hybriden Genkonstruktion in eine nur einmal vorkommende Restriktionsstelle weiter modifiziert.

Der modifizierte virale Anteil des rekombinanten Plasmids wird dann aus dem bakteriellen Eltern-Plasmid ausgeschnitten und für die Inokulation der Pflanzenzellen oder der gesamten Pflanzen verwendet.

Eine andere Methode zur Einschleusung der chimären Genkonstruktion in die Zelle bedient sich der Infektion der Pflanzenzelle mit Agrobacterium tumefaciens, welches mit besagter Genkonstruktion transformiert ist. Die transgenen Pflanzenzellen werden anschliessend unter geeigneten, dem Fachmann bekannten Kultivierungsbedingungen kultiviert, so dass sie Schösslinge und Wurzeln ausbilden und letztlich ganze Pflanzen resultieren.

Die chimäre Genkonstruktion lässt sich beispielsweise mit Hilfe des Ti-Plasmids von Agrobacterium tumefaciens in geeignete Pflanzenzellen überführen [Decleene et al., Bot. Rev., 47: 147-194 (1981); Bot. Rev., 42: 389-466 (1976)]. Das Ti-Plasmid wird im Verlaufe der Infektion durch Agrobacterium tumefaciens auf die Pflanze übertragen und stabil ins Pflanzengenom integriert. [Horsch et al.,Science, 233: 496-498 (1984); Fraley et al., Proc. Natl. Acad. Sci. USA, 80: 4803 (1983)].

Ti-Plasmide besitzen zwei Regionen, die für die Herstellung transformierter Zellen essentiell sind. Eine davon, die Transfer-DNA Region, wird auf die Pflanze übertragen und führt zur Induktion von Tumoren. Die andere, die virulenzverleihende (vir) Region, ist nur für die Ausbildung, nicht aber für die Aufrechterhaltung der Tumoren essentiell. Die Transfer-DNA Region kann in ihren Dimensionen durch Einbau der chimären Genkonstruktion vergrössert werden, ohne dass die Uebertragungsfähigkeit beeinträchtigt wird. Durch Entfernen der tumorverursachenden Gene, wodurch die transgenen Pflanzenzellen nicht-tumorös bleiben und durch Einbau eines selektionierbaren Markers, kann das modifizierte Ti-Plasmid als Vektor für den Transfer der erfindungsgemässen Genkonstruktion in eine geeignete Pflanzenzelle verwendet werden.

Die vir-Region bewirkt den Transfer der T-DNA Region von Agrobacterium auf das Genom der Pflanzenzelle, unabhängig davon, ob die T-DNA-Region und die vir-Region auf demselben Vektor oder auf verschiedenen Vektoren innerhalb derselben Agrobacterium-Zelle vorliegen. Eine vir-Region auf einem Chromosom induziert ebenso den Transfer der T-DNA von einem Vektor in eine Pflanzenzelle.

Bevorzugt wird ein System zur Uebertragung einer T-DNA-Region von einem Agrobacterium in Pflanzenzellen, das dadurch gekennzeichnet ist, dass die vir-Region und die T-DNA-Region auf verschiedenen Vektoren liegen. Ein solches System ist unter dem Begriff "binäres Vektor-System" bekannt und der die T-DNA enthaltende Vektor wird als ein "binärer Vektor" bezeichnet.

Jeder T-DNA enthaltende Vektor, der in Pflanzenzellen übertragbar ist und der eine Selektion der transformierten Zellen erlaubt, ist für die Verwendung im Rahmen dieser Erfindung geeignet.

Durch Anwendung neu entwickelter Transformationstechniken lassen sich mittlerweile in vitro auch solche Pflanzenspezies transformieren, die keine natürlichen Wirtspflanzen für Agrobacterium sind. So sind beispielsweise monokotyle Pflanzen, insbesondere die Getreidearten und Gräser, keine natürlichen Wirte für Agrobacterium.

Es gibt in der Zwischenzeit vermehrt Hinweise darauf, dass sich auch Monokotyledonen mit Agrobacterium transformieren lassen, so dass unter Verwendung von neuen experimentellen Ansätzen, die jetzt verfügbar werden, auch Getreide und Gräser-Spezies einer Transformation zugänglich sind [Grimsley NH, et al., Nature, 325: 177-179 (1987)].

Bevorzugt im Rahmen dieser Erfindung ist die direkte Einschleusung der erfindungsgemässen Genkonstruktionen in pflanzliche Protoplasten, für die ebenfalls bereits eine Reihe von Verfahren zur Verfügung stehen.

So kann das genetische Material, das in einem Vektor enthalten ist, beispielsweise mit Hilfe von Mikropipetten für den mechanischen Transfer der rekombinanten DNA direkt in die Pflanzenzellen mikroinjiziert werden (Neuhaus et al., 1987).

Weitere Möglichkeiten für den direkten Transfer von genetischem Material in die pflanzliche Zelle bestehen in der Behandlung der Protoplasten mit die Plasmamembran modifizierenden Verfahrensmassnahmen, wie z.B. der Polyethylenglykol Behandlung [Paszkowski et al., EMBO J., 3: 2717-22 (1984)], der Hitzeschockbehandlung oder der Elektroporation sowie einer Kombination dieser Verfahrensmassnahmen [Shillito et al., Bio Technology, 3: 1099-1103 (1985); Fromm et al., Proc. Natl. Acad. Sci. USA, 82: 5824 (1985)].

Bei der Elektroporations-Technik werden pflanzliche Protoplasten zusammen mit Plasmiden, die die hybride Genkonstruktion enthalten, elektrischen Impulsen hoher Feldstärke ausgesetzt. Dies führt zu einer reversiblen Permeabilitätserhöhung von Biomembranen und ermöglicht damit die Einschleusung der Plasmide. Elektroporierte pflanzliche Protoplasten erneuern ihre Zellwand, teilen sich und bilden Kallusgewebe. Die Selektion der transformierten Pflanzenzellen kann mit Hilfe der zuvor beschriebenen phenotypischen Marker erfolgen.

Ein weiteres Verfahren für die direkte Einführung von genetischem Material in Pflanzenzellen, das im Rahmen dieser Erfindung besonders bevorzugt ist, ist bei Negrutiu I et al., 1987 beschrieben.

Es handelt sich dabei um ein Verfahren, das auf rein chemischen Verfahrensmassnahmen beruht und eine sehr effiziente und schnelle Durchführung der Transformation ermöglicht.

Im einzelnen besteht dieses Verfahren darin, dass man
- pflanzliche Protoplasten aus beliebigen Pflanzengeweben isoliert und gegebenenfalls in einem der üblicherweise für die Kultivierung pflanzlicher Protoplasten verwendeten Nährmedien, kultiviert;
- besagte Protoplasten vor der eigentlichen Transformation 20 Minuten bis 6 Stunden in einem Vorinkubationsmedium, enthaltend Erdalkali und/ oder Alkalikationen, vorzugsweise $Ca^{2+}$-, K und/oder $Na^+$-Ionen sowie eine geeignete C-Quelle, bei einer Temperatur von 40 bis 10°C vorinkubiert;
- die Protoplasten anschliessend aus dem Vorinkubationsmedium abtrennt und in dem eigentlichen Transformationsmedium, enthaltend als essentiellen Bestandteil 0.1 bis 60 mM, vorzugsweise 10 bis 30 mM $Mg^{2+}$-Ionen, in An- oder Abwesenheit von $Ca^{2+}$-Ionen, resuspendiert;
- unmittelbar danach eine DNA-Probe, enthaltend ein oder mehrere Gene unter der Kontrolle von in Pflanzen aktiven Expressionssignalen sowie eine Träger-DNA, der Transformationslösung zufügt;
- einige Sekunden bis zu 20 Minuten, vorzugsweise 0,1 bis 10 Minuten, später ein die Plasmamembran modifizierendes Agenz hinzufügt;
- die Protoplasten und DNA-Probe in besagter Transformationslösung für einen Zeitraum inkubiert, der die Aufnahme der DNA in die Protoplasten gewährleistet; und
- sofern gewünscht, aus den transformierten Protoplasten ganze Pflanzen regeneriert.

Ganz besonders bevorzugt im Rahmen der vorliegenden Erfindung ist der direkte Gentransfer unter Verwendung der Co-Transformation (Schocher R.J et al., Bio/Technology, 4: 1093-1096, 1986).

Bei der Co-Transformation handelt es sich um eine Methode, die auf der gleichzeitigen Aufnahme und Integration verschiedener DNA-Moleküle (nichtselektionierbares und selektionierbares Gen) ins pflanzliche Genom beruht, und die daher das Auffinden von Zellen, die mit nicht selektierbaren Genen transformiert sind, ermöglicht.

Die zuvor gegebene beispielhafte Auflistung möglicher Transformationsverfahren erhebt keinen Anspruch auf Vollständigkeit und soll den Erfindungsgegenstand in keiner Weise beschränken.

Diejenigen Zellklone, die die hybride Genkonstruktion in ihr Genom eingebaut enthalten werden mit Hilfe üblicher Selektions-, Screening- und Nachweismethoden ausgelesen und für die Regeneration transgener Pflanzen verwendet.

Die Regeneration von in Kultur gehaltenen Protoplasten zu ganzen Pflanzen ist bei Evans, et al., "Protoplast Isolation and Culture", in Handbook of Plant Cell Culture, 1: 124-176 (Macmillan Publishing Co. New York 1983); M.R. Davey, "Recent Developments in the Culture and Regenration of Plant Protoplasts", Protoplasts, 1983 -Lecture Proceedings, Seite 19-29, (Birkhäuser, Basel 1983); P.J. Dale, "Protoplast Culture and Plant Regeneration of Cereals and Other Recalcitrant Crops", in Protoplasts 1983 - Lecture Proceedings, Seite 31-41, (Birkhäuser, Basel 1983); und H. Binding, "Regeneration of Plants", in Plant Protoplasts, Seite 21-37, (CRC Press, Boca Raton 1985) und Potryhus I and Shillito RD, Methods in Enzymology, Vol 118, Plant Molecular Biology, eds. A. and H. Weissbach, Academic Press, Orlando, 1986 beschrieben.

Die Regenerationsverfahren unterscheiden sich von Pflanzenspezies zu Pflanzenspezies. Im allgemeinen aber werden die Protoplasten in einem der bekannten Kulturmedien zur Teilung und Zellwandbildung angeregt. Es entstehen schliesslich Kalluskulturen, welche durch Behandlung mit bestimmten Wirkstoffen, wie z.B. Auxinen und Cytokininen zur Wurzel-bzw. Sprossbildung induziert werden können.

Die so gewonnenen Pflänzchen können anschliessend in Erde übertragen und in gleicher Weise wie normale Sämlinge weiterkultiviert werden.

Eine effiziente Regeneration hängt in erster Linie vom Medium, vom Genotyp und von der Vorgeschichte der Kultur ab. Werden diese drei Variablen hinreichend kontrolliert, dann ist die Regeneration vollständig reproduzier- und wiederholbar.

Die regenerierten transgenen Pflanzen, welche ein in der pflanzlichen Zelle exprimierbares Strukturgen der zuvor beschriebenen hybriden Genkonstruktion als integralen Bestandteil des pflanzlichen Genoms enthalten, werden vegetativ vermehrt vorzugsweise in Form steriler Spross-Kulturen.

Die stabile Integration eines funktionsfähigen exprimierbaren Gens ins pflanzliche Genom der regenerierten transgenen Pflanzen wird anhand der mitotischen Stabilität des integrierten Gens sowie aufgrund des Verhaltens als Mendelsches Merkmal während der Meiose und unter Verwendung der "Southern blot" Analyse (Southern EM, 1975) bestimmt.

Ein weiterer Gegenstand der vorliegenden Erfindung betrifft somit die Herstellung transgener Pflanzen mit verbesserten und/oder wünschenswerten Eigenschaften durch den gezielten Einbau von DNA an vorherbestimmbaren Stellen innerhalb des pflanzlichen Genoms, insbesondere solchen, die eine hohe Expressionseffizienz und vorteilhafte Regulierbarkeit gewährleisten.

Ebenfalls umfasst vom breiten Konzept dieser Erfindung sind transgene Pflanzen, die mit Hilfe des zuvor beschriebenen erfindungsgemässen Verfahrens transformiert worden sind sowie deren asexuelle und/oder sexuelle Nachkommenschaft, welche noch die durch die Transformation der Mutterpflanze bedingte(n) neue(n) und wünschenswerte(n) Eigenschaft(en) aufweisen.

Unter den Begriff der asexuellen und/oder sexuellen Nachkommenschaft transgener Pflanzen fallen definitionsgemäss im Rahmen dieser Erfindung somit auch alle Mutanten und Varianten die mit Hilfe bekannter Verfahren, wie z.B. durch Zellfusionen oder Mutantenselektion gewonnen werden können, und welche noch die charakteristischen Eigenschaften der transformierten Ausgangspflanze aufweisen, sowie sämtliche Kreuzungs-und Fusionsprodukte mit dem transformierten Pflanzenmaterial.

Ein weiterer Gegenstand dieser Erfindung betrifft das Vermehrungsgut transgener Pflanzen.

Unter Vermehrungsgut transgener Pflanzen soll im Rahmen dieser Erfindung jedes beliebige pflanzliche Material verstanden werden, das sich auf sexuellem oder asexuellem Wege bzw. in-vivo oder in-vitro vermehren lässt. Als besonders bevorzugt sind im Rahmen dieser Erfindung in erster Linie Protoplasten, Zellen, Kalli, Gewebe, Organe, Samen, Embryonen, Pollen, Eizellen, Zygoten anzusehen, sowie jedes beliebige andere Vermehrungsgut das von transgenen Pflanzen erhalten werden kann.

Pflanzenteile, wie z.B. Blüten, Stengel, Früchte, Blätter, Wurzeln, die von transgenen Pflanzen oder deren Nachkommen stammen, die zuvor mit Hilfe des erfindungsgemässen Verfahrens transformiert worden sind und die somit wenigstens zum Teil aus transgenen Zellen aufgebaut sind, sind ebenfalls Gegenstand dieser Erfindung.

Das erfindungsgemässe Verfahren eignet sich für die Transformation aller Pflanzen, insbesondere solchen der systematischen Gruppen Angiospermae und Gymnospermae.

Unter den Gymnospermae sind von besonderem Interesse die Pflanzen der Klasse Coniferae.

Unter den Angiospermae sind neben den Laubbäumen und Sträuchern von besonderem Interesse Pflanzen der Familien Solanaceae, Cruciferae, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae oder Gramineae und der Ordnung Leguminosae und hier vor allem der Familie Papilionaceae. Bevorzugt sind Vertreter der Familien Solanaceae, Cruciferae und Gramineae.

Zu den Zielkulturen im Rahmen der vorliegenden Erfindung zählen beispielsweise auch jene, ausgewählt aus der Reihe: Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum und Sorghum, einschliesslich derjenigen, ausgewählt aus der Reihe: Ipomoea, Passiflora, Cyclamen, Malus, Prunus, Rosa, Rubus, Populus, Santalum, Allium, Lilium, Narcissus, Ananas, Arachis, Phaseolus und Pisum.

Aufgrund neuer Enwicklungen auf dem Gebiet der in vitro Kultivierung von Pflanzen, vornehmlich im Bereich der Pflanzenregeneration, ist es inzwischen möglich, auch bei Vertretern aus der Familie der Gramineae, ausgehend von pflanzlichen Protoplasten, ganze Pflanzen zu regenerieren. Beispiele von erfolgreich durchgeführten Regenerationsexperimenten bei Gramineen sind u.a. bei Abdullah, R et al., Bio/Technology, 4: 1087-1090, 1986, Fujimura, T., et al., Plant Tissue Culture Lett, 2:74-75, 1985, Toriyama, K., et al., Theor Appl Genet, 73:16-19, 1986, Yamada, Y., et al., Plant Cell Rep, 5:85-88, 1986 (Reis) sowie bei Rhodes et al. (1988) für Maisprotoplasten beschrieben.

Es können im Rahmen der vorliegenden Erfindung daher auch die folgenden Pflanzen verwendet werden: Lolium, Zea, Triticum, Sorghum, Saccharum, Bromus, Oryzae, Avena, Hordeum, Secale und Setaria.

Die reifen Pflanzen, die aus transformierten Pflanzenzellen herangezogen wurden, werden zur Samenproduktion mit sich selbst gekreuzt. Einige der Samen enthalten die Gene, die für eine nützliche und wünschenswerte Eigenschaft kodieren in einem Verhältnis, das genau den etablierten Gesetzen der Vererbung gehorcht. Diese Samen können zur Produktion transgener Pflanzen verwendet werden.

Homozygote Linien können durch wiederholte Selbstbestäubung und Herstellung von Inzuchtlinien gewonnen werden. Diese Inzuchtlinien können dann wiederum zur Entwicklung von Hybriden verwendet werden. Bei diesem Verfahren wird eine Inzuchtlinie, die besagtes Fremdgen enthält mit einer anderen Inzuchtlinie zur Produktion gekreuzt.

Nach der allgemeinen Beschreibung der vorliegenden Erfindung soll nun zum besseren Verständnis auf spezifische Ausführungsbeispiele Bezug genommen werden, die zum Zwecke der Illustration in die Beschreibung mitaufgenommen werden, und die keinen limitierenden Charakter haben, es sei denn, es wird speziell darauf hingewiesen.

Nichtlimitierende Ausführungsbeispiele:

Allgemeine rekombinante DNA-Techniken

Da viele der in dieser Erfindung genutzten rekombinanten DNA-Techniken Routine für den Fachmann sind, ist eher hier eine kurze Beschreibung dieser allgemein gebrauchten Techniken enthalten als jedesmal dort, wo sie erscheinen. Bis darauf, wo extra darauf hingewiesen wird, sind alle diese Verfahren in der Referenz von Maniatis et al. (1982) beschrieben.

## A. Schneiden mit Restriktionsendonukleasen

Typischerweise sind etwa 50 bis 500 µg/ml DNA in dem Reaktionsansatz enthalten, in der vom Hersteller, New England Biolabs, Beverly, MA., empfohlenen Pufferlösung. 2 bis 5 Einheiten von Restriktionsendonukleasen werden für jedes µg DNA hinzugefügt und der Reaktionsansatz bei der vom Hersteller empfohlenen Temperatur für eine bis drei Stunden inkubiert. Die Reaktion wird durch 10 minütiges Erhitzen auf 65° C oder durch Extraktion mit Phenol, gefolgt durch Präzipitation der DNA mit Ethanol, beendet. Diese Technik wird auch auf den Seiten 104 bis 106 der Maniatis et al.-Referenz beschrieben.

## B. Behandlung der DNA mit Polymerase, um glatte Enden zu erzeugen

50 bis 500 µg/ml DNA-Fragmente werden zu einem Reaktionsansatz hinzugefügt, in dem vom Hersteller, Neu England Biolabs, empfohlenen Puffer. Der Reaktionsansatz enthält alle vier Desoxynukleotidtriphosphate in Konzentrationen von 0.2 mM. Die Reaktion erfolgt während 30 Minuten bei 15° C und wird dann durch 10 minütiges Erhitzen auf 65° C beendet. Für Fragmente, die durch Schneiden mit Restriktionsendonukleasen erhalten werden, welche 5′-hervortretende Enden erzeugen, wie EcoRI und BamHI, wird das grosse Fragment, oder Klenow-Fragment, der DNA-Polymerase benutzt. Für Fragmente, die durch Endonukleasen erhalten werden, welche 3′-hervortretende Enden erzeugen, wie PstI und SacI, wird die T4-DNA-Polymerase benutzt. Die Verwendung dieser zwei Enzyme wird auf den Seiten 113 bis 121 der Maniatis et al.-Referenz beschrieben.

## C. Agarose-Gelelektrophorese und Reinigung von DNA-Fragmenten von Gelen

Die Agarose-Gelelektrophorese wird in einem horizontalen Apparat durchgeführt, wie auf den Seiten 150 bis 163 der Maniatis et al.-Referenz beschrieben. Der benutzte Puffer ist der dort beschriebene Tris-Boratpuffer. Die DNA-Fragmente werden durch 0.5 µg/ml Ethidiumbromid gefärbt, das entweder im Gel- oder Tankpuffer während der Elektrophorese vorhanden ist oder nach der Elektrophorese hinzugefügt wird. Die DNA wird durch Beleuchtung mit langwelligem Ultraviolettlicht sichtbar gemacht. Wenn die Fragmente nicht vom Gel abgetrennt werden sollen, wird eine Agarose verwendet, die bei niedriger Temperatur geliert und von Sigma Chemical, St. Louis, Missouri, bezogen werden kann. Nach der Elektrophorese wird das gewünschte Fragment ausgeschnitten, in ein Plastikröhrchen gegeben, etwa 15 Minuten auf 65° C erhitzt, dreimal mit Phenol extrahiert und zweimal mit Ethanol gefällt. Dieses Verfahren ist gegenüber dem von Maniatis et al. auf Seite 170 beschriebenen leicht verändert.

Als Alternative kann die DNA aus der Agarose mit Hilfe des Geneclean Kits (Bio 101 Inc., La Jolla, CA, USA) isoliert werden.

## D. Hinzufügen von synthetischen Linkerfragmenten an DNA-Enden

Wenn es erwünscht ist, eine neue Endonukleaseschnittstelle an das Ende eines DNA-Moleküls anzufügen, wird das Molekül gegebenenfalls zuerst mit DNA-Polymerase behandelt, um glatte Enden zu erzeugen, wie im obigen Abschnitt beschrieben. Etwa 0.1 bis 1.0 µg dieses Fragments wird zu etwa 10 ng phosphorylierter Linker-DNA gegeben, die von New England Biolabs bezogen wurde, in einem Volumen von 20 bis 30 µl mit 2 µl T4 DNA-Ligase von New England Biolabs, und 1mM ATP in dem vom Hersteller empfohlenen Puffer. Nach einer Inkubation über Nacht bei 15° C wird die Reaktion durch 10 minütiges Erhitzen auf 65° C beendet. Der Reaktionsansatz wird auf etwa 100 µl in einem Puffer verdünnt, der richtig für die Restriktionsendonuklease ist, die die synthetische Linkersequenz schneidet. Ungefähr 50 bis 200 Einheiten dieser Endonuklease werden hinzugefügt. Die Mischung wird 2 bis 6 Stunden bei der angemessenen Temperatur inkubiert, dann wird das Fragment einer Agarose-Gelelektrophorese unterworfen und gereinigt wie oben beschrieben. Das resultierende Fragment wird nun Enden haben mit Endigungen, welche durch Schneiden mit der Restriktionsendonuklease erzeugt wurden. Diese Enden sind gewöhnlich kohäsiv, so dass das resultierende Fragment nun leicht an andere Fragmente mit den gleichen kohäsiven Enden geknüpft werden kann.

## E. Entfernen von 5′-terminalen Phosphaten von DNA-Fragmenten

Während der Plasmidklonierungsschritte vermindert die Behandlung des Vektorplasmids mit Phosphatase die Rezirkularisation des Vektors (diskutiert auf Seite 13 der Maniatis et al.-Referenz). Nach Schneiden der DNA mit der richtigen Restriktionsendonuklease wird eine Einheit alkalische Phosphatase aus dem Darm von Kälbern hinzugefügt, die von Boehringer-Mannheim, Mannheim, erworben wurde. Die DNA wird eine Stunde bei 37° C inkubiert und anschliessend zweimal mit Phenol extrahiert und mit Ethanol gefällt.

## F. Verknüpfen der DNA-Fragmente

Wenn Fragmente mit komplementären kohäsiven Enden miteinander verknüpft werden sollen, werden etwa 100 ng von jedem Fragment in einem Reaktionsgemisch von 20 bis 40 µl mit etwa 0.2 Einheiten T4 DNA-Ligase von New England Biolabs im vom Hersteller empfohlenen Puffer inkubiert. Die Inkubation wird 1 bis 20 Stunden lang bei 15° C durchgeführt. Wenn DNA-Fragmente mit glatten Enden verknüpft werden sollen, werden sie inkubiert wie oben, bis darauf, dass die Menge der T4 DNA-Ligase auf 2 bis 4 Einheiten erhöht wird.

## G. Die Transformation von DNA in E. coli

E. coli Stamm HB101 wird für die meisten Experimente verwendet. DNA wird mit dem Kalziumchloridverfahren, wie es von Maniatis et al., Seiten 250 bis 251, beschrieben wurde, in E. coli eingeführt.

## H. Screening von E. coli auf Plasmide

Nach der Transformation werden die resultierenden Kolonien von E. coli auf das Vorhandensein des gewünschten Plasmids durch ein schnelles Plasmidisolationsverfahren geprüft. Zwei gebräuchliche Verfahren werden auf den Seiten 366 bis 369 der Maniatis et al.-Referenz beschrieben.

I. Isolierung von Plasmid-DNA in grossem Massstab

Verfahren zur Isolierung von Plasmiden aus E. coli in grossem Massstab werden auf den Seiten 88 bis 94 der Maniatis et al.-Referenz beschrieben.

J. Klonierung in M13 Phagenvektoren

Die folgende Beschreibung ist so zu verstehen, dass die doppelsträngige replikative Form der Phage M13-Abkömmlinge für Routineverfahren, wie Schneiden mit Restriktionsendonuklease, Verknüpfen etc., benutzt wird.

Enzyme können, wenn nicht extra darauf hingewiesen wird, von Boehringer, Biolabs (BRL), bezogen werden. See werden entsprechend den Angaben des Herstellers verwendet, es sei denn, es wird gesondert darauf hingewiesen.

Beispiel 1: Konstruktion des Plasmids pABDI

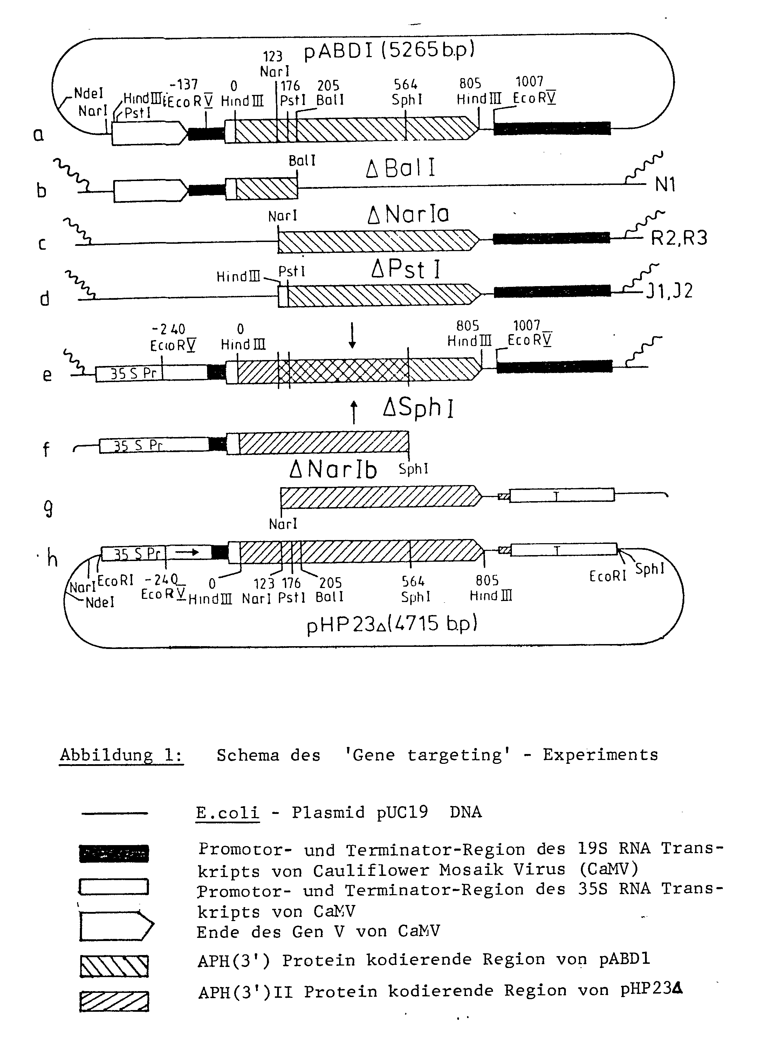

Man zerschneidet die frei zugänglichen Plasmide pkm 21 und pkm 244 [Beck, E., et al., Gene 19, 327-336 (1982)] mit der Restriktions-Endonuclease PstI. Die Fragmente der Plasmide, welche für die Rekombination verwendet werden, werden durch Elektrophorese in 0,8%igem Agarose-Gel gereinigt. Das aus der Verbindung der Bruchstücke erhaltene Plasmid pkm 21244 enthält eine Kombination der 5'-und 3'-Bal 31 - Deletionen des NPT-II-Gens, wie von Beck et al. in Gene 19, 327-336 (1982) beschrieben. Um das Promotersignal des Cauliflower-Mosaik-Virus mit dem HindIII-Fragment des Plasmids pkm 21244 zu verbinden, wird das Kupplungsplasmid pJPAK konstruiert. Das Kupplungsplasmid pJPAX wird aus den Plasmiden pUC8 und pUC9 [Messing, J. and J. Vieira, Gene 19, 269-276 (1982)] erhalten. 10 Basenpaare von der Kupplungssequenz des Plasmids pUC9 werden durch Zerschneiden bei der HindIII- und der SalI-Position und nachfolgendem Auffüllen der haftfähigen Enden mit dem Polymerase-I-Klenow-Fragment [Jacobsen, H., et al., Eur. J. Biochem. 45, 623, (1974)] und Verbinden der Polynukleotidkette, wodurch die HindIII-Position wieder hergestellt wird, herausgetrennt. Ein synthetisches Kupplungsglied von 8 Basenpaaren (XhoI) wird an der SmaI-Position dieser abgetrennten Kupplungssequenz eingefügt. Die Rekombination der geeigneten XorI- und HindIII-Fragmente des Plasmids pUC8 und des modifizierten Plasmids pUC9 ergibt das Plasmid pJPAX mit einer teilweise asymmetrischen Kupplungssequenz mit den aufeinander folgenden Restriktionsstellen: EcoRI, SmaI, BamHI, SalI, PstI, HindIII, BamHI, XhoI und EcoRI. Die CaMV Gen VI Promotor-Region, die mit dem NPT II Struktur Gen verknüpft wird, stammt aus dem Genom des CaMV-Stammes CM 4-184, einer Variante des CaMV-Stammes CM 1841, dessen komplette Nukleotidsequenz bei Gardner RC et al, 1981 beschrieben ist. Die CaMV Promotor-Region wird in dem 6 k.b umfassenden Cosmid pHC 79, einen Abkömmling des E. coli Plasmids pBR322 [Hohn B and Collins J, 1980], kloniert, wobei das CaMV Genom sowie das Cosmid pHC79 mit BstII geschnitten und die resultierenden Bruchstücke miteinander verknüpft werden. Die Verbindung des 5'-Expressionssignals des Cauliflower-Mosaik-Virus-Gens VI und des HindIII-Fragments des NPT-II-Gens wird beim Plasmid pJPAX durch Einfügung der Promoterregion des Cauliflower-Mosaik-Virus-Gens VI zwischen die PstI- und die Hind III-Position bewirkt. Das so erhaltene Plasmid wird an der einzigen HindIII-Position aufgeschnitten und das HindIII-Fragment des Plasmids pkm 21244 wird in diese Schnittstelle in beiden Orientierungsrichtungen eingebaut, wobei man die Plasmide pJPAX Cakm$^+$ und pJPAK Cakm$^-$ erhält. Um eine EcoRV-Sequenz in der Nähe des 3'Terminationssignals des NPT-II-Hybridgens zu schaffen, wird ein BamHI-Fragment des Plasmids pJPAX Cakm$^+$ in die BamHI-Position des Plasmids pBR 327 [Soberon, X. et al., Gene 9, 287-305 (1980)] eingebaut. Man erhält das Plasmid pBR 327 Cakm. Das EcoRV-Fragment des Plasmids pBR 327 Cakm, das die neue DNA-Konstruktion enthält, wird verwendet, um die EcoRV-Region des Cauliflower-Mosaik-Virus-Gens VI zu ersetzen, welches an der SalI-Position im Plasmid pUC8 kloniert ist, wodurch man die Protein-kodierende DNA-Sequenz des NPT-II-Gens unter die Kontrolle der 5'- und 3'-Expressionssignale des Cauliflower-Mosaik-Gens VI stellt. Die so hergestellten Plasmide tragen die Bezeichnung pABDI und PABDII (vgl. Abbildung 1).

Beispiel 2: Konstruktion des Plasmids pHP23Δ

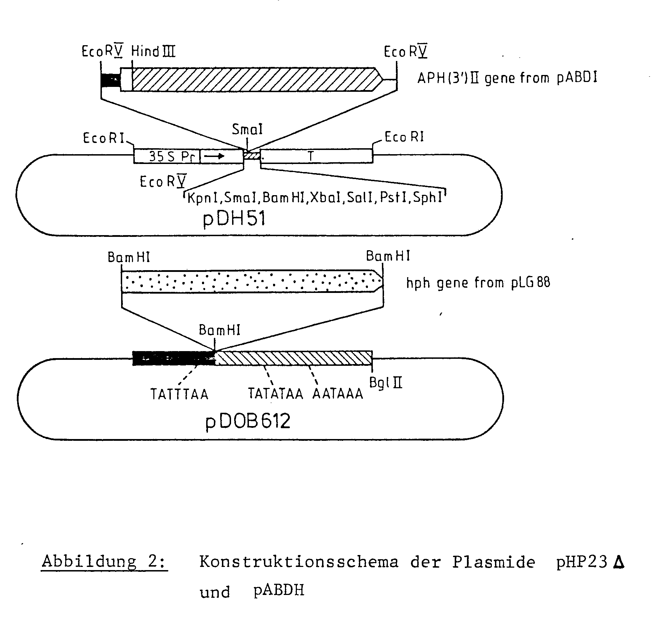

Die Konstruktion von Plasmid pHP23 erfolgt durch Insertion eines EcoRV-Fragments des Plasmids pABD1, welches das APH(3')II-Strukturgen sowie einen Teil der 19S RNA Promotor-Sequenz von CaMV enthält, in die SmaI-Seite des Plasmids pDH51 (vgl. Abbildung 2).

Das Plasmid pDH51 ist bei Pietrzak et al., 1986 beschrieben. Dieses Plasmid enthält die 35S Promotor-Region und Terminator-Region von CaMV, die getrennt sind durch eine eingeschobene Polylinker-Region, die zahlreiche Klonierungsstellen enthält.

Als Ausgangsmaterial für die 35S Promotor-/Terminator Region dient ein ScaI-Fragment von CaMV "S", das die Nukleotide 6808-7632 der Genkarte (Frank G et al., Cell, 21: 285-294, 1980) umfasst, und das in die SmaI-Stelle von pUC18 (Norrander J. et al., Gene, 26: 101-106, 1983) eingespleisst wird, unter Bildung von Plasmid pDB21. Dies wird anschliessend mit dem Restriktionsenzym HphI verdaut. Die kohesiven Enden werden durch Behandlung mit T4 DNA Polymerase entfernt.

Ein Fragment, welches die 35S Promotor-Region enthält [pUC18 pos. 21 (HphI)-412 (SmaI) plus CaMV pos. 6808-7437 (HphI)] wird mit KpnI Linkern verknüpft, mit NcoI (pos. 6909 CaMV) geschnitten, die kohesiven Enden mit Hilfe des Klenow-Fragments der DNA-Polymerase aufgefüllt, mit EcoRI-Linkern verknüpft und schliesslich mit EcoRI und KpnI verdaut.

Das resultierende EcoRI/KpnI-Fragment, das die CaMV Promotor-Sequenz enthält wird dann in das Plasmid pUC18 zwischen die KpnI und EcoRI Restriktionsschnittstellen eingespleisst. Man erhält auf diese Weise das Plasmid pDG2.

Ein zweites Fragment, das die Terminationssignale trägt [CaMV pos. 7439 (HphI)-7632 plus pUC18 pos. 413-1550 (HphI)], wird mit EcoRI geschnitten, die kohesiven Enden werden mit Klenow aufgefüllt, mit HindIII Linkern versehen und mit HindIII und SphI verdaut.

Das resultierende SphI-HindIII Fragment, das die CaMV Terminator-Sequenz enthält, wird dann in das Plasmid pDG2 eingespleisst und zwar in die HindIII und SphI Restriktionsschnittstellen.

pHP23 wird modifiziert durch Deletion eines Teils der Polylinker-Region. Nach Schneiden des Plasmids mit BamHI und SphI und Deletion des BamHI-SphI Fragments werden die kohäsiven Enden mit Hilfe der T4-Polymerase entfernt und die Bruchstücke wieder miteinander verknüpft unter Bildung von pHP23$\Delta$.

Die Konstruktion von pHP23$\Delta$ wird komplettiert durch Reklonierung eines EcoRI-Fragmentes, welches das Hybrid-Gen trägt, im Plasmid pUC19 (Roberts RJ, 1984), was die Herstellung von Deletionsmutanten erleichtert.

## Beispiel 3: Konstruktion des Plasmids pABDH

Das BamH1 Fragment von Plasmid pLG88, das bei Blochlinger und Diggelmann, Mol. Cell. Biol., 4(12): 2929 - 2931, 1984 beschrieben ist und das die Protein kodierende Region der Hygromycinphosphotransferase (hph) enthält, wird in die BamHI Seite vom pDOB612 (Balazs E et al., Gene, 4O: 343-348, 1985) eingespleisst unter Bildung von pABDH (vgl. Abbildung 2).

## Beispiel 4: Gen-targeting

2 Plasmide mit verschiedenen Konstrukten des APH(3') II hybriden Marker-Gens werden für die Herstellung von sich gegenseitig komplementierenden Deletionsmutanten verwendet.

### N$_1$-Stamm:

1. Plasmid pABD1 (s. Abb. 1a) beschrieben bei Paszkowski J et al., Embo J, 3, 2717-2722, 1984), wird mit den Restriktionsenzymen BalI und SmaI geschnitten. Durch Deletion des BalI-SmaI-Fragments von pABD1 erhält man die Deletionsmutante $\Delta$-BalI (s. Abb. Ib).

2. Linearisierung dieses Plasmids an der NdeI Restriktionsschnittstelle.

3. Integration des linearisierten Plasmids ins nucleare Genom von Tabak durch Co-Transformation mit pABDH. Man erhält auf diese Weise den N$_1$-Stamm des Target-Tabak.

### R$_2$, R$_3$-Stamm

1. Schneiden von pABDI mit NarI. Durch Deletion des NarI-Fragments von Plasmid pABDI erhält man die Deletionsmutante $\Delta$ NarIa (s. Abb. 1c).

2. Linearisierung des Plasmids an der NdeI Restriktionsschnittstelle.

3. Co-Transformation von $\Delta$NarI in das Tabak-Genom. Man erhält die Target-Stämme R$_2$ und R$_3$.

### J$_1$, J$_2$-Stamm

1. Schneiden von pABDI mit PstI. Durch Deletion des PstI-Fragments von Plasmid pABDI erhält man die Deletionsmutante $\Delta$Pst I (s. Abb. 1d).

2. Linearisierung des Plasmids an der NdeI Restriktionsschnittstelle.

3. Einbringen von $\Delta$PstI ins Tabak Genom liefert die Target Stämme J$_1$ und J$_2$.

### Deletions-Mutanten

#### $\Delta$SphI (p19SphI):

Schneiden von Plasmid pHP23$\Delta$ mit SphI und Deletion des SphI-Fragments führt zu der Deletionsmutanten $\Delta$SphI (s. Abb. If).

#### $\Delta$NarIb (p19NarIb):

Schneiden von Plasmid pHP23b mit NarI und Deletion des NarI-Fragments führt zu der Deletionsmutanten $\Delta$NarIb (s. Abb. Ig).

Die verwendeten Methoden für die Plasmid-Konstruktion sind bei Maniatis et al., 1982 beschrieben.

## Beispiel 5: Herstellung von Tabakprotoplasten

Tabakprotoplassten werden nach herkömmlichen Verfahren ausgehend von einer Tabak-Suspensionskultur hergestellt (Potrykus I and Shillito RD, Methods in Enzymology, Vol. 118, Plant Molecular Biology, eds. A and H. Weissbach, Academic Press, Orlande, 1986). Vollständig entfaltete Blätter von 6-Wochen alten Spross-Kulturen werden unter sterilen Bedingungen entfernt und mit einer Enzymlösung der folgenden Zusammensetzung gründlich benetzt.

Enzymlösung:

| | |
|---|---|
| H$_2$O | 70 ml |
| Saccharose | 13 g |
| Macerozyme | 1 g |
| R 10 | |
| Cellulose | 2 g |
| 'Onozuka' R$_{10}$ | |
| Drisellase | 0.13 g |
| MES | 0.5 ml |
| pH | 6.0 |

Man zerschneidet die Blätter anschliessend in 1-2 cm grosse quadratische Stücke und lässt sie auf der oben genannten Enzymlösung aufschwimmen. Die Inkubation erfolgt über Nacht bei einer Temperatur von 26° C im Dunkeln. Dieser Ansatz wird anschliessend leicht geschwenkt und für weitere 30 min bis zur vollständigen Verdauung inkubiert.

Diese Suspension wird dann zur Abtrennung unverdauter Gewebereste durch ein Stahlsieb mit einer Maschenweite von 100 $\mu$m filtriert, mit 0.6 M Sucroselösung (MES, pH 5-6) durchgespült und anschliessend 10 Minuten bei 100 g zentrifugiert. Aufgrund dieser Behandlung sammeln sich die Protoplasten an der Oberfläche des Mediums, das dann unter den Protoplasten abgezogen wird, z.B. unter Verwendung einer sterilisierten Injektionsspritze.

Die Protoplasten werden anschliessend in einem K3A Medium resuspendiert, das 0,4 M Sucrose enthält und zur Entfernung von anhaftendem Enzym 3 x durch Flotation und anschliessendes Entfernen des Mediums, in der zuvor beschriebenen Weise, gewaschen wird. Die gereinigten Protoplasten werden schliesslich in einer Lösung von 0,4 M Mannit und 1 g/Liter MES derart suspendiert, dass eine Protoplastendichte von 1,2 bis 1,6 x $10^4$ ml vorliegt.

Beispiel 6: Co-Transformation von Protoplasten von Nicotiana tabacum c.v. Petit Havanna SRI mit Hilfe der Mg$^{2+}$/PEG-Behandlung (Negrutiu, I, et al, 1987)

Die co-Transformation von Deletionsplasmid-DNA und pABDH-DNA wird entsprechend den Anweisungen von Schocher et al, 1986, durchgeführt. Zur Durchführung der Transformationsexperimente werden die Protoplasten zunächst gewaschen, gezählt und anschliessend in einem W5-Medium [154 mM NaCl, 125 mM CaCl$_2$ 2H$_2$O, 5mM KCl, 5 mM Glucose, pH 5.6; Menczel, L. et al. (1981)], das eine hohe Ueberlebensrate der isolierten Protoplasten gewährleistet, in einer Zelldichte von 1-2.5 x $10^6$ Zellen pro ml resuspendiert. Die Protoplasten werden anschliessend nach einer Inkubationszeit von 30 Minuten bei 6°C bis 8°C für die Transformationsexperimente verwendet.

Kurz vor der eigentlichen Transformation der isolierten Protoplasten wird das W5-Medium durch das eigentliche Transformationsmedium ersetzt. Es handelt sich dabei um eine Mannit/Magnesium-Lösung [MaMg-Lsg: 0.4-0.5 mM Mannit, 0.1 % MES (Morpholinethansulfonsäure), pH 5.6] mit einer Mg$^{2+}$-Konzentration von 12 bis 16 mM. Dazu werden die Protoplasten zunächst aus der W5-Lösung durch 5-minütige Zentrifugation bei 100 g abgetrennt und in dem MaMg-Medium resuspendiert (0.3 ml). Zu dieser Suspension werden dann 65 µl einer wässrigen DNA-Lösung, enthaltend 5 - 10 µg Deletionsplasmid-DNA, 2 µg des Plasmids pABDH und 40 µg Kalbsthymus-Carrier DNA, zugesetzt. Bei letzterer handelt es sich um eine neutrale Carrier-DNA ohne transformierendes Insert, die zum Schutz vor einer Nukleaseverdauung der zu transformierenden DNA zu diesem Ansatz im Ueberschuss hinzugegeben wird. Plasmid und Carrier DNA werden zuvor durch Ethanol Reprezipitation sterilisiert, wie bei Paszkowski und Saul, 1986, beschrieben. Nach einer Zeitspanne von ca. 0.1 bis 10 Minuten nach der DNA-Zugabe erfolgt die Applikation einer wässrigen 40 %igen Polyethylenglykollösung (w/v) bis eine Endkonzentration von 24 bis 28% (w/v) erreicht ist. Als besonders vorteilhaft erweist sich die Verwendung von PEG vom CMS Typ. Dabei handelt es sich um eine Ca$^{2+}$-haltige [0.1 M Ca(NO$_3$)$_2$•4H$_2$O] 0.4 M Mannitlösung, die PEG vom Molekulargewicht ca. 1000 bis ca. 6000 in einer Endkonzentration von 40 % (w/v) enthält. Der pH-Wert dieser Lösung liegt bei pH 7 bis 9 [Negrutiu, I. et al. (1986a)]

Im Falle von Nicotiana tabacum c.v. Petit Havanna SRI verwendet man vorzugsweise PEG CMS 4000. Der pH-Wert des Transformationsmediums wird anschliessend auf einen Wert von pH 7 eingestellt. Diese Mischung wird unter gelegentlichem Umschwenken für 30 Minuten bei 26°C inkubiert.

Bei Verwendung von hohen PEG-Konzentrationen von >20% ist eine stufenweise Verdünnung mit dem 3 bis 5-fachen Volumen einer 0.2 M CaCl$_2$-Lösung vorteilhaft. Anschliessend werden die in der angegebenen Weise behandelten Protoplasten abzentrifugiert (5 Minuten bei 100 g) und in frischem K$_3$-Medium resuspendiert (0.3 ml Protoplastenlösung in 10 ml frisches K$_3$-Medium). Die weitere Inkubation erfolgt in 10 ml-Portionen in Petri-Schalen von 10 cm Durchmesser bei 24°C und Dunkelheit, wobei die Populationsdichte 4 bis 8x$10^4$ Protoplasten pro ml beträgt. Nach 3 Tagen wird das Kulturmedium pro Schale mit 0.3 Volumenteilen frischem K$_3$-Medium verdünnt und für weitere 4 Tage bei 24°C und 3000 lux künstlicher Beleuchtung inkubiert. Nach insgesamt 7 Tagen werden die aus den Protoplasten entwickelten Klone in mit 1 % Agarose verfestigtem, 12 mg/l Hygromycin enthaltendem Nährmedium eingebettet und nach der "bead type"-Kulturmethode [Shillito, R.D. et al., Plant Cell Reports, 2, 244-247 (1983)] bei 24°C unter Dunkelheit kultiviert. Das Nährmedium wird alle 5 Tage durch frische gleichartige Nährlösung ersetzt.

Nach Ablauf von 6 Wochen werden die Hygromycin-resistenten Klone, die einen Durchmesser von 1-2 mm erreicht haben auf ein mit Agar verfestigtes antibiotikahaltiges Medium ohne Hygromycin überführt.

Die Kultivierungsbedingungen sowie das Regenerations-Protokoll sind bei Saul et al., 1987, beschrieben.

Beispiel 7: Untersuchung Hygromycin-resistenter Klone auf den Einbau von APH(3')II Deletionsmutanten

Insgesamt wurden 7 Hygromycin-resistente Klone mit Hilfe der Southern blot Hybridisation auf den Einbau von APH(3')II Deletionsmutanten in das Pflanzen-Genom überprüft.

Fünf der untersuchten Klone, die die erwartete Genkonstruktion besitzen, (3 veschiedene APH(3')II-Gen-Deletionen) werden für die weiteren Experimente verwendet.

Die Kopienzahl der target-Deletionen variiert in einem Bereich von zwei bis zehn. (Stamm J$_1$ - 3 Kopien, Stamm J$_2$ - 7 Kopien. Stamm N$_1$ - 5 Kopien, Stamm R$_2$ - 2 Kopien, Stamm R$_3$ - 10 Kopien).

Von diesen Zellinien werden dann transgene Pflanzen gemäss dem bei Saul et al., 1987 beschriebenen Regenerationsprotokol regeneriert und in Form steriler Sprosskulturen weitervermehrt.

Nach mehreren Subkultivierungen erhält man Blätter, die eine gute Ausbeute an proliferierenden Protoplasten liefern.

Die Integration der Genkonstrukte in das nukleare Genom wird an Hand der mitotischen Stabilität und der Vererbung als ein singuläres Mendel'sches Merkmal mit Hilfe der Southern blot Hybridisierung verifiziert.

Beispiel 8: "Gen Targeting" Experimente

Für die "gene targeting" Experimente werden Protoplasten von den 5 zuvor ausgewählten Linien verwendet. Plasmid DNA, die komplementierende Abschnitte des chimären APH(3') II - Gens enthält, wird mit Hilfe des im Beispiel 6 beschriebenen direkten Gentransfer-Verfahrens in die Protoplasten der korrespondierenden Pflanzenstämme eingeschleust, wobei die folgenden Kombinationen gewählt werden.

p19NarIb mit Stamm $N_1$

p19SphI mit Stamm $R_2$, $R_3$, $J_1$ und $J_2$.

Beispiel 9: Auslese Kanamycin-resistenter Zellklone

Nach 3 bis 4 Wochen fortgesetzter Kultivierung in Kanamycin-haltigem Nährmedium werden die resistenten Kalli von 2 bis 3 mm Durchmesser auf mit Agar verfestigtes LS-Kulturmedium [Linsmaier EM and Skoog F, Physiol. Plant. 18: 100-127, 1965], enthaltend 0.05 mg/l 2,4-Dichlorphenoxyessigsäure, 2 mg/l 1-Naphthylessigsäure, 0.1 mg/l 6-Benzylaminopurin, 0.1 mg/l Kinetin und 75 mg/l Kanamycin, verpflanzt. Durch Sprossinduktion auf LS-Medium, enthaltend 150 mg/l Kanamycin und 0.2 mg/l 6-Benzylaminopurin, und anschliessender Wurzelbildung auf T-Medium [Nitsch, YP and Nitsch C, Science, 163: 85-87 1969] erhält man Kanamycin-resistente Nicotiana tabacum-Pflanzen der Sorte Petit Havanna SRI.

Beispiel 10: Nachweis des NPT-II-Gens im Pflanzenerbgut

0,5 g Kallus der transformierten Zellkulturen oder Blattgewebe der daraus regenerierten Pflanzen werden bei 0°C in 15 %-iger Saccharoselösung, enthaltend 50 mMol/l Ethylendiamin-N,N,N',N'-tetraessig-säure, EDTA), 0,25 Mol/l Natriumchlorid und 50 mMol/l $\alpha,\alpha,\alpha$-Tris-(hydroxymethyl)methylamin-Hydrochlorid (TRIS-HCl) bei pH 8,0 homogenisiert. Durch Zentrifugieren des Homogenisats für 5 Minuten bei 1000 g trennt man grob die Zellkerne ab. Die Zellkerne werden in 15 %-iger Saccharoselösung, enthaltend 50 mMol/l EDTA und 50 mMol/l TRIS-HCl, bei pH 8,0 resuspendiert, mit Natrium-dodecylsulfat bis zu einer Endkonzentration von 0,2 % versetzt und 10 Minuten auf 70°C erhitzt. Nach Abkühlung auf 20-25°C wird der Mischung Kaliumacetat bis zu einer Konzentration von 0,5 Mol/l zugesetzt. Dieses Gemisch wird 1 Stunde bei 0°C inkubiert. Der ausgefallene Niederschlag wird durch Zentrifugieren (15 Minuten bei 4°C in einer Mikrozentrifuge) zentrifugiert. Aus der überstehenden Flüssigkeit wird durch Versetzen mit dem 2,5-fachen Volumen Ethanol bei 20-25°C die DNS ausgefällt. Die abgetrennte DNS wird in einer Lösung von 10 mMol TRIS-HCl, enthaltend 10 μg/ml Ribonuclease A, gelöst, 10 Minuten bei 37°C inkubiert, mit Proteinase K bis zu einer Konzentration von 250 μg/ml versetzt und eine weitere Stunde bei 37°C inkubiert. Die Proteinase K wird durch Phenol- und Chloroform/Isoamylalkohol-Extraktionen entfernt. Aus der wässrigen Phase wird die DNS durch Zusatz von 0,6 Volumenteilen 0,6 molarer isopropanolischer Natriumacetat-Lösung ausgefällt und in 50 μl einer Lösung, enthaltend 10 mMol/l TRIS-HCl und 5 mMol/l EDTA, bei pH 7,5 gelöst. Durch diese Präparation erhält man DNS-Sequenzen, die vorwiegend mehr als 50'000 Basenpaare enthalten. Restriktion dieser DNA mit EcoRV-Endonuclease, Hybridisierung der Fragmente mit radioaktiv markierten HindIII-Bruchstücken des NPT-II-Gens und Vergleich mit dem Plasmid pABDI zeigt in einer "Southern Blot"-Analyse die Anwesenheit des NPT-II-Gens in der Zellkern-DNS der transformierten Nicotiana tabacum-Zellen.

Beispiel 11: Southern blot-Analyse:

Die Analyse der Pflanzen DNA wird entsprechend einer bei Paszkowski und Saul, 1983, beschriebenen Methode, durchgeführt.

Ungefähr 5 μg genomischer DNA werden wahlweise mit EcoRV oder HindIII-Restriktionsenzymen geschnitten und die erhaltenen Bruchstücke durch Elektrophorese in einem 1 % Standard-Agarose-Gel aufgetrennt. (Maniatis T et al., 1982).

Die Southern blot Analyse wird gemäss den Angaben von Paszkowski und Saul, 1986, durchgeführt.

Die Nick-Translation wird dabei ersetzt durch die "random primer" Methode, die bei Feinberg und Vogelstein, 1983, beschrieben ist.

Dies führt zu einer reproduzierbaren Aktivität von mehr als $10^8$ Cpm/μg DNA.

Ergebnisse

Aus 3 unabhängig voneinander durchgeführten Experimenten, an denen insgesamt, 1,4 x $10^8$ Protoplasten beteiligt waren, konnten 8 Kanamycin resistente Zellklone erhalten werden.

In den gleichzeitig durchgeführten Kontrollversuchen, bei denen Wildtyp SR1 Protoplasten verwendet wurden, und bei denen keine komplementären Deletionsmutanten angeboten wurden, konnten keine resistenten Klone beobachtet werden.

Kanamycin-resistente Zellinien können in erster Linie von den Empfängerstämmen $J_2$ und $R_3$ erhalten werden.

Die Frequenz der Bildung Kanamycin-resistenter Klone ist vergleichbar mit der Transformationsfrequenz von parallel durchgeführten Versuchen, bei denen nicht-unterbrochene hybride Genkonstruktionen verwendet wurden.

Die verwendeten 3' und 5' APH(3')II Gen-Deletionen stammen von zwei hybridene Genen, die sich in ihrer Promotor und Terminator-Region unterscheiden.

Daher führt eine homologe Rekombination dieser Deletionsmutanten zu einem Markergen, das neu kombinierte flankierende DNA Sequenzen trägt, die zu andersartigen Restriktionsfragmenten führen im Vergleich zu den zuvor existierenden Genkonstrukten.

Die Southern blot Analyse bestätigt, dass eine homologe Rekombination an den zentralen Homologiebereichen der zwei APH(3')II Deletionsmutanten stattgefunden hat und schliesst damit gleichzeitig die Möglichkeit einer Transformation durch kontaminierende Eltern-Plasmide aus.

## DNA-Analyse

Die DNA von 4 J₂ komplementierten Klonen wurde untersucht.

Ein EcoRV-Fragment (1247 bp), das im Zusammenhang mit dem neuen Marker-Gen steht, konnte bei sämtlichen untersuchten Kanamycin-resistenten Klonen nachgewiesen werden.

Gleichzeitig macht die Southern blot Analyse deutlich, dass nicht alle integrierten APH(3')II Gendeletionen korrigiert wurden.

Das Erscheinen von DNA-Fragmenten, die der wiederhergestellten vollständigen Gen-Kopie entsprechen, geht einher mit einer Abnahme der deletierten Gen-Kopien. Dies ist als ein Hinweis darauf zu werten, dass es innerhalb des Genoms durch homologe Rekombination zu einer spezifischen Korrektur der mutierten Gen-Kopie durch die eingeschleuste DNA kommt.

Eine genomische Genkartierung zeigt, dass ein Hind III Fragment von 805 bp, das die Protein kodierende Region des APH(3')II Gens trägt, exakt rekonstruiert wird.

Dies bedeutet, dass mit hoher Wahrscheinlichkeit die hybride Genkorrektur exakt abläuft.

## Nachweis der Uebertragung des transformierten Gens auf die sexuellen Nachkommenschaften sowie seiner Vererbung als normales Pflanzengen

Umfangreiche genetische Kreuzungsanalysen und ausführliche molekularbiologische Studien (beispielsweise "Southern blot"-Analyse der DNS des Pflanzengenoms; Untersuchung der Enzymaktivität der Aminoglykosidphosphotransferase, d.h., dem Enzym für die Kanamycin-spezifische Phosphorylierung) mit den genetisch veränderten Pflanzen (erste Generation und Nachkommenschaften) haben folgende Ergebnisse erbracht:

1. Das bakterielle Gen ist stabil in das Pflanzengenom eingebaut;

2. Es wird in der Regel unverändert und regelmässig auf Kreuzungsnachkommenschaften übertragen;

3. Sein Erbgang entspricht dem eines natürlichen, einfachen, dominanten Pflanzengens;

4. Die molekulare Analyse mittels DNS-Hybridisierung und Enzymtest bestätigt die Ergebnisse der genetischen Kreuzungsanalyse;

5. Die genetisch veränderten Pflanzen haben ihren normalen, natürlichen Phänotyp während der Behandlung beibehalten; es sind also keine unerwünschten Veränderungen festgestellt worden.

Diese Ergebnisse zeigen, dass mit dem erfindungsgemässen Verfahren des direkten Gentransfers in Protoplasten der beste Weg für die gezielte genetische Veränderung von pflanzlichem Material aufgefunden worden ist. Die genetische Veränderung ist stabil, und unerwünschte Aenderungen des Genotyps der Pflanze treten nicht auf.

Aus der nach a) erhaltenen Kanamycin-resistenten Tabakpflanzen wird nach an sich bekannten Methoden, nämlich durch Selbstung und Auslese eine homozygote Linie gewonnen. Damit stehen Nicotiana-Tabacum Pflanzen zur Verfügung, bei denen jedes haploide Genom nur eine einzige Kopie des Neomycintransferase-Gens NPT-II enthält.

Hinterlegung:

Das im Rahmen der vorliegenden Erfindung verwendete Plasmid p19SphI wurde bei der als Internationale Hinterlegungsstelle anerkannten 'Deutschen Sammlung von Mikroorganismen' (DSM), Bundesrepublik Deutschland, entsprechend den Anforderungen des Budapester Vertrages für die Internationale Anerkennung der Hinterlegung von Mikroorganismen zum Zwecke der Patentierung, hinterlegt. Eine Erklärung zur Lebensfähigkeit der hinterlegten Proben wurde durch die besagte Internationale Hinterlegungsstelle ausgefertigt.

| Mikroorganismus | Hinterlegungsdatum | Hinterlegungs-Nummer* | Datum der Lebensfähigkeits-Bescheinigung |
|---|---|---|---|
| p19SphI (Escherichia coli transformiert mit p19SphI Plasmid DNA | 27.10.1987 | DSM 4289 | 4.11.1987 |

* Hinterlegungs-Nummer, ausgegeben durch die oben bezeichnete Internationale Hinterlegungsstelle.

## Literaturverzeichnis:

1. Abdullah R et al., Bio/Technology, 4: 1087-1090, 1986

2. Ambros PF et al., EMBO J., 5: 2073-2077, 1986.

3. Balazs E et al., Gene, 40: 343-348, 1985

4. Beck E et al., Gene, 19: 327-336, 1982

5. Berry-Lowe et al., J. Mol. Appl. Genet. 1, 483-498, 1982.

6. Binding H, "Regeneration of Plants", in Plant Protoplasts, Seite 21-37, (CRC Press, Boca Raton 1985)

7. Blochlinger und Diggelmann, Mol. Cell. Biol., 4(12): 2929-2931, 1984

8. Cashmore A, Genetic Engineering of Plants, an Agricultural Perspectiv, Plenum Press, New York, 1983, pp 29-38

9. Coruzzi G et al. The Journal of Biological Chemistry, 258: 1399, 1983

10. Dale PJ, "Protoplast Culture and Plant regeneration of Cereals and Other Recalcitrant Crops", in Protoplasts 1983 - Lecture Proceedings, Seiten 31-41, (Birkhäuser Basel, 1983).

11. Davey MR, "Recent Developments in the Culture of Plant Protoplasts", Protoplasts, 1983 - Lecture Proceedings, Seite 19-29, (Birkhäuser, Basel 1983);

12a. De Clene et al., Bot. Rev., 47: 147-194, 1981,

12b. De Clene et al., Bot. Rev., 42: 389-466, 1976,

13. De Lozanne A and Spudich JA, Science, 236: 1086-1091, 1987

14. Dunsmuir P et al., J. Mol. Appl. Genet., 2: 285, 1983

15. Evans, et al., "Protoplast Isolation and Culture", in Handbook of Plant Cell Culture, 1: 124-176 (Macmillan Publishing Co. New York 1983)

16. Feinberg AP and Vogelstein B, Analytical Biochemistry, 132: 6-13, 1983

17. Fraley et al., Proc. Natl. Acad. Sci. USA, 80: 4803, 1983

18. Frank G et al., Cell, 21: 285-294, 1980

19. Fromm et al., Proc. Natl. Acad. Sci. USA, 82: 5824, 1985

20. Fujimura T, et al., Plant tissue Culture Lett, 2: 74-75, 1985

21. Gardner RC et al., Nucl. Acids Res., 9: 2871-2888, 1981

22. Gressel J, Oxford Surv. Plant Mol. Cell Biol., 2: 321 - 328, 1985

23. Gritz I and Davies J, Gene, 25: 179-188, 1983

24. Hinnen H et al., Proc. Natl. Acad. Sci, USA, 75: 1929-1933, 1978

25. Hohn et al, in "Molecular Biology of Plant Tumors" Academic Press, New York, Seite 549-560, 1982

26. Hohn B and Collins J, Gene, 11: 291-298, 1980

27. Horsch et al., Science, 233: 496-498, 1984

28. Howell, US-Patent No. 4,407,956

29. Jacobsen H et al., Eur. J. Biochem., 45: 623, 1974

30. Linsmaier EM and Skoog F, Physiol. Plant, 18: 100-127, 1965

31. Maniatis et al., Molecular Cloning, Cold Spring Harbor Laboratory, 1982

32. Menczel L et al., Theor. Appl. Genet., 59: 191-195, 1981

33. Messing J and Vieira J, Gene, 19: 269-276: 1982

34. Miller BI et al., Mol. Cell. Biol., 7: 427-434, 1987

35. Negrutiu J. et al., Plant Mol. Biol., 8: 363-373, 1987

36. Negrutiu J. et al., Theor. Appl. Genet, 72: 279-286, 1986a

37. Neuhaus G. et al., Theor. Appl. Genet., 74: 30-36, 1987

38. Nitsch YP and Nitsch C, Science, 163: 85-87, 1969

39. Norrander J et al., Gene, 26: 101-106, 1983

40. Paszkowski J et al., EMBO J., 3: 2717-2722, 1984

41. Paszkowski J and Saul M, Methods in Enzymology, 118: 627-646, 1986

42. Pietrzak M. et al., Nucl. Acids Res., 14: 5857-5868, 1986

43. Potrykus J et al., Mol. Gen. Genet., 199: 169-177, 1985

44. Potrykus I and Shillito RD, Methods in Enzymology, Vol 118, Plant Molecular Biology, eds. A. and H. Weissbach, Academic Press, Orlando, 1986.

45. Rhodes el al., Biotechnology, 6: 56-59, 1988

46. Roberts RJ, Nucleic Acids Res., 12, 1984

47. Schocher RJ et al., Bio/Technology, 4: 1093-1096, 1986

48. Shillito RD, et al., Bio/Technology, 3: 1099-1103, 1985

49. Shillito RD, et al., Plant Cell Reports, 2: 244-247, 1983

50. Soberon X et al., Gene, 9: 287-305, 1980

51. Southern EM, J. Mol. Biol., 98: 503-517, 1975

52. Toriyama K, et al., Theor Appl Genet, 73: 16-19, 1986

53. Wallroth M et al., Mol. Gen. Genet, 202: 6-15, 1986

54. Wienand K et al., Mol. Gen. Genet, 182: 440-444, 1981

55. Yamada Y, et al., Plant Cell Rep, 5: 85-88, 1986.

56. Zarawski G et al, Proc. Natl. Acad. Sci, USA, 79: 7699 - 7703, 1982

**Patentansprüche**

1. Rekombinantes DNA Molekül, welches eine zielgerichtete Integration von Genen, Genfragmenten oder sonstigen DNA-Sequenzen an genau definierten Stellen innerhalb des pflanzlichen Genoms ermöglicht, dadurch gekennzeichnet, dass besagtes rekombinantes DNA Molekül die zu integrierende DNA enthält, und das besagte DNA in einem Masse Homologien zu entsprechenden DNA Regionen innerhalb des pflanzlichen Genoms aufweist oder aber von solchen homologen DNA-Sequenzen flankiert wird, dass bei der Transformation der die homologen DNA Regionen enthaltenden pflanzlichen Zelle eine zielgerichtete Integration besagter zu integrierender DNA an genau definierten Stellen innerhalb des pflanzlichen Genoms durch homologe Rekombination stattfindet.

2. Rekombinantes DNA Molekül gemäss Anspruch 1, dadurch gekennzeichnet, dass die zu integrierenden DNA, die gegebenenfalls unter der Kontrolle von in der pflanzlichen Zelle funktionsfähigen Expressionssignalen steht, mit flankierenden DNA-Abschnitten, die Homologien zu einer bestimmten Region innerhalb des pflanzlichen Genoms aufweisen, verknüpft ist.

3. Rekombinantes DNA Molekül gemäss Anspruch 1, dadurch gekennzeichnet, dass die zu integrierende DNA, die gegebenenfalls unter der Kontrolle von in der pflanzlichen Zelle funktionsfähigen Expressionssignalen steht, eine genügend grosse Homologie zu entsprechenden DNA-Regionen innerhalb des pflanzlichen Genoms aufweist, sodass ein direkter Austausch besagter zu integrierender DNA gegen besagte homologe genomische DNA vermittels der homologen Rekombination erfolgt.

4. Rekombinantes DNA-Molekül gemäss einem der Ansprüche 1 bis 3, dadurch gekennzeichnet, dass besagte zu integrierende DNA entweder aus genomischer DNA, aus einer cDNA oder aus synthetischer DNA besteht.

5. Rekombinantes DNA-Molekül gemäss einem der Ansprüche 1 bis 3, dadurch gekennzeichnet, dass besagte zu integrierende DNA sowohl aus genomischer DNA, als auch aus cDNA und/oder synthetischer DNA zusammengesetzt ist.

6. Rekombinantes DNA-Molekül gemäss einem der Ansprüche 1 bis 3, dadurch gekennzeichnet, dass die zu integrierende DNA aus Gen oder sonstigen DNA-Fragmenten von mehreren Organismen, die verschiedenen Gattungen angehören, zusammengesetzt ist.

7. Rekombinantes DNA-Molekül gemäss einem der Ansprüche 1 bis 3, dadurch gekennzeichnet, dass die zu integrierende DNA aus Gen oder sonstigen DNA-Fragmenten von Organismen, die verschiedenen Stämmen, oder Varietäten einer Art oder verschiedenen Spezies einer Gattung angehören, zusammengesetzt ist.

8. Rekombinantes DNA-Molekül gemäss einem der Ansprüche 1 bis 3, dadurch gekennzeichnet, dass die zu integrierende DNA aus Anteilen von mehr als einem Gen des selben Organismus zusammengesetzt ist.

9. Rekombinantes DNA Molekül gemäss einem der Ansprüche 1 bis 3, dadurch gekennzeichnet, dass es sich bei besagter zu integrierender DNA um ein Strukturgen handelt, das der transformierten Pflanze eine nützliche und wünschenswerte Eigenschaft verleiht.

10. Rekombinantes DNA Molekül gemäss Anspruch 9, dadurch gekennzeichnet, dass besagtes Strukturgen der Pflanze eine erhöhte Resistenz oder Toleranz gegenüber Pathogenen, Herbiziden, Insektiziden oder anderen Bioziden verleiht.

11. Rekombinantes DNA Molekül gemäss Anspruch 9, dadurch gekennzeichnet, dass besagtes Strukturgen die Bildung und Qualität von Reserve- und Lagerstoffen in Blättern, Samen, Knollen, Wurzeln und Stengeln verbessert.

12. Rekombinantes DNA Molekül gemäss Anspruch 11, dadurch gekennzeichnet, dass es sich bei besagtem Strukturgen um pflanzliche Speicherproteingene handelt.

13. Rekombinantes DNA Molekül gemäss Anspruch 9, dadurch gekennzeichnet, dass besagtes Strukturgen für pharmazeutisch akzeptable Wirksubstanzen kodiert.

14. Rekombinantes DNA Molekül gemäss Anspruch 1, dadurch gekennzeichnet, dass es sich bei der zu integrierenden DNA um nicht-kodierende DNA-Sequenzen mit regulatorischer Funktion handelt.

15. Rekombinantes DNA Molekül gemäss Anspruch 2, dadurch gekennzeichnet, dass besagte Expressionssignale aus Genen von Pflanzen oder Pflanzenviren stammen.

16. Rekombinantes DNA Molekül gemäss Anspruch 15, dadurch gekennzeichnet, dass besagte Expressionssignale aus einem pflanzlichen Gen stammen, das für die kleine Untereinheit der Ribulose-I,5-bisphosphatcarboxylase oder das Chlorophyll a/b Bindungsprotein kodiert.

17. Rekombinantes DNA Molekül gemäss Anspruch 15, dadurch gekennzeichnet, dass besagte Expressionsignale aus Genen von Pflanzenviren stammen.

18. Rekombinantes DNA Molekül gemäss Anspruch 17, dadurch gekennzeichnet, dass es sich um Cauliflower Mosaik Virus (CaMV) handelt.

19. Rekombinantes DNA Molekül gemäss Anspruch 18, dadurch gekennzeichnet, dass es sich um die 35 S-Expressionssignale des CaMV Genoms handelt.

2O. Rekombinantes DNA Molekül gemäss Anspruch 18, dadurch gekennzeichnet, dass es sich um die 19 S Expressionssignale des CaMV-Genomes handelt.

21. Rekombinantes DNA-Molekül gemäss Anspruch 1, dadurch gekennzeichnet, dass es sich bei besagten flankierenden DNA-Sequenzen um solche handelt, die Homologien zu pflanzeneigenen Genen oder Genomabschnitten aufweisen.

22. Rekombinantes DNA Molekül gemäss Anspruch 1, dadurch gekennzeichnet, dass es sich bei besagten flankierenden DNA-Sequenzen um solche handelt, die Homologien zu Gerien oder Genfragmenten oder zu sonstigen nützlichen DNA-Sequenzen aufweisen, welche zuvor mit Hilfe bekannter Verfahrensmassnahmen artifiziell ins Genom einer Zielpflanze integriert wurden und deren Nukleotidsequenzen ganz oder teilweise bekannt sind.

23. Rekombinantes DNA Molekül gemass Anspruch 22, dadurch gekennzeichnet, dass es sich bei besagter flankierender DNA und der artifiziell ins Pflanzengenom integrierten DNA um sich gegenseitig komplementierende DNA-Sequenzen handelt, die nach erfolgter homologer Rekombination eine funktionelle Einheit innerhalb des pflanzlichen Genoms bilden.

24. Rekombinantes DNA Molekül gemass Anspruch 23, dadurch gekennzeichnet, dass die sich gegenseitig komplementierenden DNA-Sequenzen nach erfolgter Rekombination einen phänotypischen Marker oder sonstige leicht detektierbare Proteinprodukte kodieren.

25. Rekombinantes DNA Molekül gemäss einem der Ansprüche 21 bis 24, dadurch gekennzeichnet, dass besagte Gene oder Genfragmente oder sonstige nützliche DNA-Sequenzen in Bereichen innerhalb des pflanzlichen Genoms lokalisiert sind, die natürlicherweise eine hohe Expressionsrate aufweisen.

26. Rekombinantes DNA Molekül gemäss Anspruch 21, dadurch gekennzeichnet, dass es sich um pflanzliche Speicherproteingene handelt.

27. Rekombinantes DNA Molekül gemäss Anspruch 1, dadurch gekennzeichnet, dass

a) besagtes rekombinantes DNA Molekül ein Strukturgen gemäss einem der Ansprüche 9 bis 14 enthält

b) besagtes Strukturgen gegebenenfalls in operabler Weise mit in pflanzlichen Zellen funktionsfähigen Expressionssignalen gemäss einem der Ansprüche 15 bis 20 verknüpft ist

c) besagte funktionelle Einheit, bestehend aus Strukturgen und gegebenenfalls Expressionssignalen in einer Weise von DNA-Sequenzen flankiert wird, die in einem Masse Homologien zu entsprechenden DNA-Regionen innerhalb des pflanzlichen Genoms aufweisen, dass bei der Transformation der die besagte homologen Region enthaltenden pflanzlichen Zelle eine gezielte Integration der von homologen DNA-Sequenzen flankierten Gensequenz an definierten Stellen innerhalb des pflanzlichen Genoms durch homologe Rekombination stattfindet.

28. Verfahren zur Herstellung transgener Pflanzen, dadurch gekennzeichnet, dass man ein Gen, ein Genfragment oder sonstige nützliche DNA Sequenzen unter Verwendung eines rekombinanten DNA Moleküls gemäss einem der Ansprüche 1 bis 27 mit Hilfe bekannter Verfahren in eine pflanzliche Zelle transformiert und dort via homologer Rekombination gezielt an einer gewunschten, vorherbestimmbaren Stelle in das pflanzliche Genom integriert.

29. Verfahren gemäss Anspruch 28, dadurch gekennzeichnet, dass die Transformation des die homologen DNA-Abschnitte enthaltenden pflanzlichen Materials unter Verwendung des Agrobacterium-Transformationssystems durchgeführt wird.

30. Verfahren gemäss Anspruch 28, dadurch gekennzeichnet, dass besagte Transformation unter Verwendung des CaMV-Transformationssystems durchgeführt wird.

31. Verfahren gemäss Anspruch 28, dadurch gekennzeichnet, dass besagte Transformation durch direkten Gentransfer erfolgt.

32. Verfahren gemäss Anspruch 28, dadurch gekennzeichnet, dass es sich bei besagter Transformation um eine Co-Transformation handelt.

33. Verfahren gemäss Anspruch 28, dadurch gekennzeichnet, dass man natürliche oder modifizierte Gene oder Genfragmente oder sonstige nützliche DNA-Sequenze durch Rekombination mit homologen DNA-Abschnitten innerhalb des pflanzlichen Genoms in dieses integriert.

34. Verfahren gemäss Anspruch 28, dadurch gekennzeichnet, dass die Integration in einer Region des pflanzlichen Genoms erfolgt, die eine hohe Expressionseffizienz und/oder eine vorteilhafte Regulierbarkeit des integrierten Gens gewährleistet.

35. Verfahren gemäss Anspruch 33, dadurch gekennzeichnet, dass besagte homologe genomische DNA-Abschnitte ein natürlicher Bestandteil des Pflanzengenoms sind.

36. Verfahren gemäss Anspruch 31, dadurch gekennzeichnet, dass besagte homologe genomische DNA-Abschnitte artifiziell mit Hilfe bekannter Verfahrensmassnahmen ins Pflanzengenom integriert worden sind.

37. Verfahren gemäss Anspruch 36, dadurch gekennzeichnet, dass besagte homologe genomische DNA-Abschnitte heterologen Ursprungs sind.

38. Verfahren gemäss einem der Ansprüche 33 bis 37, dadurch gekennzeichnet, dass besagte homologe genomische DNA-Abschnitte in genomischen Regionen mit hoher Expressionseffizienz vorliegen.

39. Verfahren gemäss einem der Ansprüche 36 oder 37, dadurch gekennzeichnet, dass besagte homologe genomische DNA-Abschnitte für einen phänotypischen Marker oder andere leicht detektierbare Proteinprodukte kodieren.

40. Ein DNA-Transfer-Vektor, dadurch gekennzeichnet, dass er ein rekombinantes DNA-Molekül gemäss einem der Ansprüche 1 bis 27 enthält.

41. Ein DNA-Expressions-Vektor, dadurch gekennzeichnet, dass er ein rekombinantes DNA-Molekül gemäss einem der Ansprüche 1 bis 27 enthält.

42. Eine Wirtszelle, dadurch gekennzeichnet, dass sie einen DNA-Transfer-Vektor gemäss Anspruch 40 enthält.

43. Eine Wirtszelle, dadurch gekennzeichnet, dass sie einen DNA-Expressions-Vektor gemäss Anspruch 41 enthält.

44. Eine Wirtszelle gemäss einem der Ansprüche 42 oder 43, dadurch gekennzeichnet, dass sie ein rekombinantes DNA-Molekül gemäss einem der Ansprüche 1 bis 27 enthält.

45. Eine Wirtszelle gemäss einem der Ansprüche 42 oder 43, dadurch gekennzeichnet, dass es sich dabei um einen Mikroorganismus handelt.

46. Eine Wirtszelle gemäss einem der Ansprüche 42 oder 43, dadurch gekennzeichnet, dass es sich dabei um eine pflanzliche Zelle handelt.

47. Pflanzliches Material bestehend aus einer transgenen Pflanze und deren Samen, dadurch gekennzeichnet, dass besagtes pflanzliches Material mit einem rekombinanten DNA Molekül gemäss einem der Ansprüche 1 bis 27 transformiert worden ist.

48. Eine pflanzliche Zelle, dadurch gekennzeichnet, dass sie ein rekombinantes DNA-Molekül gemäss einem der Ansprüche 1 bis 27 enthält.

49. Pflanzliches Material bestehend aus einer transgenen Pflanze und deren Samen, dadurch gekennzeichnet, dass besagtes pflanzliches Material pflanzliche Zellen gemäss Anspruch 48 enthält.

50. Pflanzliches Material bestehend aus einer transgenen Pflanze und deren Samen, dadurch gekennzeichnet, dass besagtes pflanzliches Material aus pflanzlichen Zellen gemäss Anspruch 48 regeneriert worden ist.

51. Pflanzliches Material gemäss einem der Ansprüche 47, 49 oder 50 ausgewählt aus der Gruppe bestehend aus Solanaceae, Cruciferae, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae, Gramineae oder Leguminosae.

52. Pflanzliches Material gemäss einem der Ansprüche 47, 49 oder 50 ausgewählt aus der Gruppe Solanaceae, Cruciferae und Gramineae.

53. Pflanzliches Material gemäss einem der Ansprüche 47, 49 oder 50, ausgewählt aus der Gruppe bestehend aus Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum und Sorghum, einschliesslich derjenigen, ausgewählt aus der Reihe: Ipomoea, Passiflora, Cyclamen, Malus, Prunus, Rosa, Rubus, Populus, Santalum, Allium, Lilium, Narcissus, Ananas, Arachis, Phaseolus und Pisum.

54. Pflanzliches Material gemass einem der Ansprüche 47, 49 oder 50, ausgewählt aus der Gruppe Lolium, Zea, Triticum, Sorghum, Saccharum, Bromus, Oryzae, Avena, Hordeum, Secale und Setaria.

55. Nachkommen einer transgenen Pflanze gemäss einem der Ansprüche 47 und 49 bis 54, wobei besagte Nachkommenschaft Mutanten und Varianten miteinschliesst.

56. Vermehrungsgut transgener Pflanzen gemäss einem der Ansprüche 47 und 49 bis 55, dadurch gekennzeichnet, dass es sich um Protoplasten, Zellen, Kalli, Gewebe, Organe, Samen, Pollen, Eizellen, Zygoten, Embryonen oder anderes von einer transgenen Pflanze erhältliches Vermehrungsgut handelt.

57. Pflanzenteile, wie z.B. Blüten, Stengel, Früchte, Blätter, Wurzeln, die von transgenen Pflanzen oder deren Nachkommen stammen, die zuvor mit Hilfe des erfindungsgemässen Verfahrens transformiert worden sind und die somit wenigstens zum Teil aus transgenen Zellen aufgebaut sind.

Abbildung 1:    Schema des  'Gene targeting' - Experiments

────────    E.coli - Plasmid pUC19  DNA

Promotor- und Terminator-Region des 19S RNA Transkripts von Cauliflower Mosaik Virus (CaMV)

Promotor- und Terminator-Region des 35S RNA Transkripts von CaMV

Ende des Gen V von CaMV

APH(3') Protein kodierende Region von pABD1

APH(3')II Protein kodierende Region von pHP23Δ

EcoRⅤ HindⅢ                                  EcoRⅤ
                                                        APH(3')Ⅱ gene from pABDI

EcoRI                    SmaI              EcoRI
        35 S Pr →              T
EcoRⅤ
Kpnl,SmaI,BamHI,XbaI,SalI,PstI,SphI
                pDH51

BamHI                        BamHI
                                                        hph gene from pLG88

BamHI

TATTTAA        TATATAA AATAAA        BglⅡ
                pDOB612

Abbildung 2:    Konstruktionsschema der Plasmide  pHP23 Δ
                und   pABDH